(19) Europäisches Patentamt
European Patent Office
Office européen des brevets

(11) Publication number: **0 211 490 B1**

(12) **EUROPEAN PATENT SPECIFICATION**

(45) Date of publication of patent specification: **26.02.92**

(51) Int. Cl.[5]: **C07K 9/00**, A61K 37/02, C12P 21/04, //(C12P21/04, C12R1:01)

(21) Application number: **86304649.6**

(22) Date of filing: **17.06.86**

(54) **Antibiotics of the vancomycin-class.**

(30) Priority: **20.06.85 US 746688**

(43) Date of publication of application:
**25.02.87 Bulletin 87/09**

(45) Publication of the grant of the patent:
**26.02.92 Bulletin 92/09**

(84) Designated Contracting States:
**AT BE CH DE FR GB IT LI LU NL SE**

(56) References cited:
**EP-A- 0 132 116**

**JOURNAL OF THE AMERICAN CHEMICAL SOCIETY, vol. 106, no. 17, 22nd August 1984, pages 4891-4895, American Chemical Society; A.H. HUNT et al.: "Structure of the major glycopeptide of the teicoplanin complex"**

(73) Proprietor: **SMITHKLINE BEECHAM CORPORATION**
**One Franklin Plaza P.O. Box 7929**
**Philadelphia Pennsylvania 19103(US)**

(72) Inventor: **Sitrin, Robert David**
**2076 Sierra Road**
**Plymouth Meeting Pennsylvania 19462(US)**

(74) Representative: **Waters, David Martin, Dr. et al**
**Smith Kline & French Laboratories Ltd. Patent Department Mundells**
**Welwyn Garden City Hertfordshire AL7 1EY(GB)**

**EP 0 211 490 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

AAD 216 antibiotics of the vancomycin-class are produced by cultivating the new microorganism, Kibdelosporangium aridum Shearer, gen. nov., sp. nov. SK&F AAD 216 (ATCC 39323) in aqueous nutrient medium, containing assimilable sources of carbon and nitrogen, under submerged aerobic conditions until a substantial amount of the AAD 216 antibiotic complex is produced. The AAD 216A, B and C antibiotics and the microorganism, K. aridum, are disclosed in European Patent Application No. 132,118, which is incorporated herein by reference as though fully set forth. The aglycone and the pseudoaglycones of AAD 216A, B and C are disclosed in European Patent Application No. 132,116, which is incorporated herein by reference as though fully set forth.

BRIEF DESCRIPTION OF THE FIGURE

The Figure is an HPLC chromatograph of the AAD 216 complex, the peaks representing factors within the complex, prepared as described in Example 8.

SUMMARY OF THE INVENTION

This invention relates to novel AAD 216 factors which are found within the AAD 216 complex disclosed in European Patent Application No. 132,118. More particularly, the invention is an AAD 216 antibiotic having the following structural formula

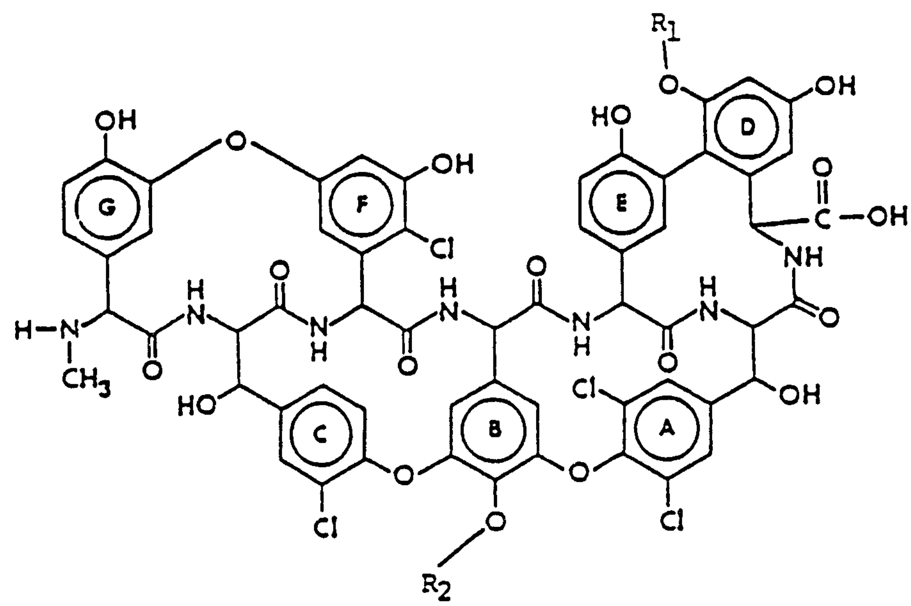

(I)

or a desoxy analog thereof wherein $R_1$ is mannosyl and $R_2$ is

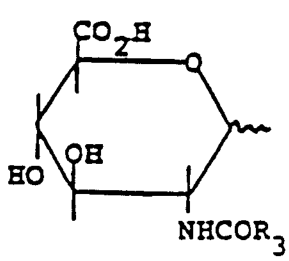

(II)

wherein $R_3$ is a $C_{8-12}$ alkyl or alkenyl side chain which may be branched or linear and which is optionally

substituted by a hydroxyl group provided that $R_3$ is different than the corresponding side chain in AAD 216A, AAD 216B and AAD 216C.

This invention is also pseudoaglycones of such AAD 216 antibiotics wherein $R_1$ is -H.

This invention is also the desoxy mannosyl pseudoaglycone ($R_1$ is mannosyl and $R_2$ is hydrogen) and the desoxy aglycone ($R_1$ is hydrogen and $R_2$ is hydrogen).

The compounds of this invention exhibit antibacterial activity and are useful in animal health applications such as growth promotion, treatment of bovine mastitis and improvement in milk production in ruminants.

## DETAILED DESCRIPTION OF THE INVENTION

The AAD 216 complex is produced by the fermentation of Kibdelosporangium aridum Shearer gen. nov., sp. nov. SK&F-AAD 216. The above microorganism was isolated from a soil sample collected in a desert area in Pima County, Arizona. A culture of the biologically pure microorganism has been deposited in the American Type Culture Collection, Rockville, Maryland as the type culture under accession number ATCC 39323.

The AAD 216 complex refers to the mixture of the individual antibiotics produced by the fermentation of K. aridum. As is readily understood by those familiar with antibiotics fermentation processes, the ratios of the individual antibiotics, or factors, and their presence in the AAD 216 complex will vary, depending on the conditions of the fermentation process. The AAD 216 complex may be recovered from the fermentation medium by clarifying the whole fermentation broth by filtration and by precipitating the crude AAD 216 complex at 0 to 10° C with hydrochloric acid at pH 3. The precipitate is then dissolved in water by adjusting the pH to about 6 to 8 and applied to a resin column from which it is eluted with aqueous methanol or acetonitrile. The resultant eluant is lyophilized to yield the AAD 216 complex which is chromatographed to afford an enriched AAD 216 complex.

The individual pure antibiotic components of the AAD 216 complex can be isolated from the AAD 216 complex utilizing preparative high pressure liquid chromatography (HPLC). The appropriate fractions, as determined by analytical HPLC, are combined and desalted on a resin column and after lyophilization afford the desired individual antibiotics.

## THE MICROORGANISM

The microorganism, Kibdelosporangium aridum, is described in EP-A-132,118. Stock cultures of Kibdelosporangium aridum SK&F-AAD 216 (ATCC 39323) were maintained on thin potato-carrot or oatmeal agar. Morphological observations were made on plates of water agar, thin potato-carrot agar, oatmeal agar and soil extract agar. Inoculum for the biochemical and physiological tests was prepared by adding the contents of a frozen vial of vegetative culture to a flask of glucose-yeast extract broth which was placed on a rotary shaker at 28° C, 250 RPM for three to six days. The culture was harvested by centrifugation and washed three times with sterile distilled water. Incubation temperature for the biochemical and physiological tests was 28° C. Readings of the results were made at various times up to 21 days for the plate media. Most of the tubed media were read at various times up to 28 days. However, the tests for decomposition of urea, allantoin and hippurate, as well as the tests for reduction of nitrates, were read for six weeks.

Susceptibility of K. aridum to antibiotics was examined by placing BBL susceptibility disks on nutrient agar plates seeded with K. aridum as an overlay. Diameters of the zones of inhibition were measured after incubation for one week at 28° C.

Morphology. K. aridum is a filamentous organism that forms a mycelium differentiated into: (1) a substrate mycelium that penetrates the agar and forms a compact layer on top of the agar, and (2) an aerial mycelium that bears chains of conidia and/or sporangium-like structures. No motile elements were observed in the aerial or substrate mycelium. On many media, K. aridum produces characteristic crystals in the agar.

Substrate Mycelium. K. aridum produces a well developed substrate mycelium which may undergo fragmentation without displacement. The long branching hyphae are septate and about 0.4 µm-1.0 µm in diameter. Present on the substrate mycelium are specialized structures which consist of dichotomously branched, septate hyphae radiating from a common stalk.

Aerial Mycelium. The aerial mycelium of K. aridum, produces chains of rod-shaped, smooth walled spores which are irregular in length (0.4 µm x 0.8 µm -2.8 µm). These spore chains are usually very long with more than 50 spores per chain, but a few short chains of ten spores or less are also usually present. The spore chains may be born apically or on short lateral branches.

On most media, the aerial mycelium of K. aridum also produces sporangium-like structures. These may

be born apically or on short lateral branches. Sporangium-like structures and chains of spores may be born on the same aerial hyphae. At maturity these sporangium-like structures are round, approximately 9 µm - 22 µm in diameter, and consist of septate, branched hyphae embedded in an amorphous matrix which is surrounded by a well-defined wall. When placed on agar, these sporangium-like structures germinate directly with the production of one or more germ tubes.

Chemotaxonomy. Purified cell wall preparations of K. aridum analyzed by the method of Becker [Becker et al. Appl. Microbiol. 12, 421-23 (1964)] contained the meso-isomer of 2,6-diaminopimelic acid, alanine, glutamic acid, glucosamine and muramic acid as well as galactose and a very minor amount of arabinose. Whole-cell hydrolysates analyzed by the method of Lechevalier [Lechevalier, M.P., J. Lab. Clin. Med. 71, 934-44 (1968)] contained galactose, glucose, mannose, ribose, rhamnose and arabinose; traces of madurose may also be present. No mycolic acids of any type were present in the cell extracts analyzed for lipid patterns by the method of Lechevalier [Lechevalier et al., Can. J. Microbiol. 19, 965-72 (1973)]. The phospholipids present were phosphatidyl ethanolamine, phosphatidyl methylethanolamine, diphosphatidyl glycerol, phosphatidyl inositol and phosphatidyl inositol mannosides. Thus K. aridum has a Type IV cell wall with a whole-cell sugar pattern of Type A with traces of madurose [Lechevalier et al., Int. J. Syst. Bacteriol. 20, 435-43 (1970)] and a phospholipid pattern of type PII with phosphatidyl methylethanolamine [Lechevalier et al., Biochem. System. Ecol. 5, 249-60 (1977)].

Physiological and Biochemical Characteristics. K. aridum is gram positive and not acid-fast. No growth takes place under anaerobic conditions. Temperature range for growth is 15°C to 42°C with a trace of growth at 45°C. Growth at 10°C is inconsistent. No growth occurs at 50°C. Hydrogen sulfide is produced. Milk is peptonized. Gelatin is both hydrolyzed and liquified. Nitrate is not reduced to nitrite. Melanin pigments are produced. Casein, L-tyrosine, hypoxanthine, guanine, elastin and testosterone are hydrolyzed but adenine, xanthine and cellulose (Avicel) are not. Catalase and phosphatase are produced. Urea, esculin and hippurate are decomposed; tests for allantoin decomposition are weakly positive. There is no growth in 8% NaCl; growth in 5% to 7% NaCl is inconsistent. No growth occurs in lysozyme broth. Acid is produced from L-arabinose, D-cellobiose, dextrin, dextrose, D-fructose, glycerol, glycogen, D-galactose, i-inositol, lactose, D-mannitol, D-mannose, α-methyl-D-glucoside, α-methyl-D-mannoside, melibiose, D-melezitose, raffinose, rhamnose, D-ribose, sucrose, trehalose, D-xylose and maltose. No acid is produced from dulcitol, i-erythritol, inulin, D-sorbitol, or L-sorbose. Citrate, malate, succinate, oxalate, lactate, acetate, pyruvate, propionate and formate are utilized; benzoate and tartrate are not.

Susceptibility to Antibiotics. By the diffusion method, K. aridum was resistant to disks impregnated with gentamicin (10 µg), tobramycin (10 µg), streptomycin (10 µg), vancomycin (30 µg), penicillin (10 units), bacitracin (2 units), lincomycin (2 µg), clindamycin (2 µg), and cephalothin (30 µg). Chlortetracycline (5 µg) produced 18 mm zones of inhibition; tetracycline (5 µg) 11-12 mm zones; rifampin (5 µg) 11-15 mm zones; novobiocin (5 µg) 27-28 mm zones. All zones of inhibition contained at least a few resistant colonies.

Description of K. aridum on Various Media

All cultures were incubated at 28°C in closed petri dish cans and observed at intervals up to 21 days. The colors of the culture were chosen by comparison with color chips from either the ISCC-NBS Centroid Color Charts or the Dictionary of Color [Maerz, A. and M. R. Paul 2nd. ed. New York: McGraw Hill Book Co., Inc. (1950)].

Yeast Extract-Malt Extract Agar - Growth excellent; vegetative growth grayish yellow brown; aerial mycelium, none to very sparse, white, spore chains and sporangium-like structures present; yellow brown soluble pigment; characteristic crystals present in agar.

Oatmeal Agar - Growth good; vegetative growth off-white to yellow brown; aerial mycelium sparse, white, numerous spore chains and sporangium-like bodies present; no soluble pigment; characteristic crystals present in agar.

Glycerol-Asparagine Agar - Growth fair to good; vegetative growth pale yellow brown; aerial mycelium sparse to moderate, white, a few spore chains but few, if any, sporangium-like structures present; pale grayish yellow brown soluble pigment; characteristic crystals present in agar.

Inorganic-Salts Starch Agar - Growth good; vegetative growth off-white to yellow brown; aerial mycelium sparse, white, spore chains and sporangium-like structures present; no soluble pigment; characteristic crystals present in agar.

Czapek-Sucrose Agar - Growth good; vegetative growth off-white to yellow brown; aerial mycelium sparse, white, spore chains and sporangium-like structures present; pale grayish yellow to pale yellow brown soluble pigment present; characteristic crystals present in agar.

Bennett's Agar - Growth good to excellent; vegetative growth grayish yellow brown; aerial mycelium,

none to sparse, white, spore chains and sporangium-like structures present; yellow brown soluble pigment; no crystals detected in agar.

Nutrient Agar - Growth fair to good; vegetative growth yellow brown; aerial mycelium sparse to moderate, white, few spore chains and few sporangium-like structures present; yellow brown soluble pigment; characteristic crystals variably present in agar.

Thin Potato-Carrot Agar - Growth fair, relatively flat; vegetative growth off-white to yellow brown; aerial mycelium sparse to moderate, white, numerous spore chains and sporangium-like structures present; no soluble pigment; no crystals detected in agar.

Peptone-Yeast Extract Iron Agar - Growth good; vegetative growth bronze brown (Maerz & Paul 16C9); aerial mycelium, none; dark brownish black soluble pigment; no crystals detected in agar.

Starch-Casein Nitrate Agar - Growth good; vegetative mycelium off-white to yellow brown; aerial mycelium sparse, white, spore chains and sporangium-like structures present; pale yellow brown soluble pigment variably present; characteristic crystals present in agar.

Yeast Extract-Glucose Agar - Growth good to excellent; vegetative growth dark yellow brown to grayish yellow brown; aerial mycelium, none visible, under 400X sparse spore chains but no sporangium-like structures present; dark yellow brown soluble pigment; characteristic crystals present in agar.

A comparison of the description of K. aridum with the descriptions of actinomycetes listed in Bergey's Manual of Determinative Bacteriology, the Approved List of Bacterial Names and other recent taxonomic literature indicates that K. aridum differs significantly from previously described species of actinomycetes and cannot be accommodated in any of the previously described genera of the actinomycetes. Sporangia, where present in other actinomycete genera, are true spore vesicles; at maturity they contain aplanospores or zoospores which are eventually released by rupture or dissolution of the sporangial wall. Despite extensive observation and manipulation, the release of spores from the sporangium-like structuress of K. aridum was never observed. When placed on agar, the sporangium-like structures of K. aridum germinate by the production of one or more germ tubes directly from the sporangium-like structure.

Type culture ATCC 39323 is herein described as a species of a new genus Kibdelosporangium aridum, (from kibdelos, Gr. adj., false, ambiguous; spora Gr. n., a seed; angium Gr. n., a vessel); the specific epithet, aridum, (aridus, L. adj., dry, arid) refers to the desert soil from which the culture was isolated.

PREPARATION OF THE AAD 216 COMPLEX

The AAD 216 complex may be produced by cultivating a strain of Kibdelosporangium having the characteristics of ATCC 39323 or an active mutant or derivative thereof, obtained by procedures known to the art, under submerged aerobic conditions in an aqueous nutrient medium. The organism is grown in a nutrient medium containing an assimilable carbon source, for example an assimilable carbohydrate. Examples of suitable carbon sources include sucrose, lactose, maltose, mannose, fructose, glucose, and soluble starch. The nutrient medium should also contain an assimilable nitrogen source such as fish meal, peptone, soybean flour, peanut meal, cotton seed meal or corn steep liquor. Nutrient inorganic salts can also be incorporated in the medium. Such salts may comprise any of the usual salts capable of providing sodium, potassium, ammonium, calcium, phosphate, sulfate, chloride, bromide, nitrate, carbonate or like ions.

Production of the AAD 216 complex can be effected at any temperature conducive to satisfactory growth of the organism, e.g., 15°-42° C., and is conveniently carried out at a temperature of about 25° to 28° C.

The medium normally is neutral, but the exact pH can be varied between 5.0 and 9.0 depending on the particular medium used.

The fermentation may be carried out in Erlenmeyer flasks or in laboratory or industrial fermentors of various capacities. When tank fermentation is to be used, it is desirable to produce a vegetative inoculum in a nutrient broth by inoculating a small volume of the culture medium with the vegetative cells of the organism. After obtaining an inoculum in this manner, it is transfered aseptically to the fermentation tank medium for large scale production of the antibiotics. The medium used for the vegetative inoculum can be the same as that employed for larger fermentations, although other media can be employed.

As is customary in aerobic submerged culture processes, sterile air is sparged through the culture medium. Agitation may be maintained by means of agitators generally familar to those in the fermentation industry.

In general, optimum production of the complex is achieved after incubation periods of about 144-186 hours in stir-jar fermentors or tank fermentors. The course of the fermentation can be followed by analytical HPLC.

The AAD 216 complex so produced contains the AAD 216 antibiotics of this invention as well as AAD 216A, AAD 216B and AAD 216C. The AAD 216 factors of this invention have the same core aglycone as factors A, B and C or a desoxy analog thereof but differ from the A, B, and C antibiotics in the fatty acid side chain ($R_3$) and, in some cases, by having one less oxygen in the core structure, that is, in the mannosyl pseudoaglycone. The desoxy analogs appear to lack the benzylic hydroxyl group in ring C in formula (I). The glycolipid moiety has been assigned the following structure:

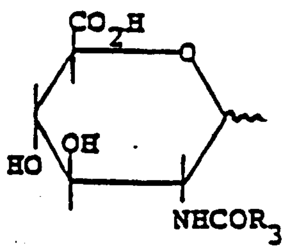

(II)

wherein $R_3$ is the side chain. $R_3$ is $C_{8-12}$ branched or linear alkyl and, in some cases, is olefinic or diolefinic and, in some cases, contains a hydroxyl group.

The Figure illustrates a HPLC chromatograph of the AAD 216 complex obtained from a representative fermentation broth substantially by the procedure of Example 8, below. The peaks in the chromatograph which are known to correspond to AAD 216 antibiotics are identified by letter designation. The lipid moiety of each purified antibiotic was analyzed by gas chromatography (GC) of the methyl ester of the fatty acid. The fatty acid esters were prepared as follows. Three mg of each AAD 216 antibiotic was dissolved in 1.0 ml of methanolic hydrochloric acid (conc. $HCl/CH_3OH$, 1/5 v/v). The solution was heated to reflux in a sealed Pierce reaction tube with a 110° C bath. After 4 hours heating, the solution was cooled to room temperature (20-25° C), and treated (in the reaction tube) with 2.0 ml of 5% sodium bicarbonate solution. The neutralized solution was extracted twice with 0.5 ml of pentane. The combined pentane extract was dried over anhydrous $Na_2SO_4$, and the solution was carefully transferred into a sample vial after filtering through cotton (not under vacuum). Each sample was analyzed in a Quadrex glass capillary, Type 007-OV17 column (25 m x 0.25 mm) in an oven programmed from 60° C to 240° C at a rate of 10° C per minute. Detection was by flame ionization and mass spectrometry.

The following table lists the antibiotics identified in the Figure (other than the first peak, E, which is the pseudoaglycone) and shows the HPLC retention times (in minutes) of the antibiotics (See, Figure) and the gas chromatography retention times (GC-RT) (in minutes) of the methyl esters of the fatty acid side chains. The table also shows the molecular weights by GC mass spectroscopy of the methyl esters. Redundant masses are due to branched isomers. In the cases of factors which comprised mixtures of factors, namely, F, G, L, M, P, B3-4 and C5-6 the approximate relative amount of each is reported as a percentage of the total (%).

| Factor | HPLC RT | GC-RT | MW | % |
|---|---|---|---|---|
| F1 | 5.9 | 11.8 | 216 | 40 |
| 2 | | 11.9 | 214 | 20 |
| 3 | | 12.1 | 216 | 40 |
| G1 | 7.7 | 10.8 | 212 | 18 |
| 2 | | 12.9 | 230 | 23 |
| 3 | | 13.0 | 230 | 59 |
| H | 8.4 | 13.8 | 230 | |
| J | 8.7 | 13.3 | 230 | |
| K | 9.1 | 13.8 | 230 | |
| L1 | 9.2 | 13.3 | 230 | 15 |
| 2 | | 13.6 | 230 | 36 |
| 3 | | 13.8 | 228 | 35 |
| M1 | 10.1 | 14.1 | 244 | 20 |
| 2 | | 14.1 | 244 | 20 |
| 3 | | 13.3 | 230 | 26 |
| N | 10.6 | 14.1 | 244 | |
| O | 10.7 | 14.6 | 244 | |
| P1 | 11.4 | 14.5 | 244 | 42 |
| 2 | | 9.7 | 202 | 30 |
| 3 | | 6.5 | 172 | 24 |
| Q | 11.7 | 9.7 | 202 | |
| D | 12.8 | 7.9 | 184 | |
| R | 13.6 | 8.2 | 184 | |
| S | 14.0 | 7.4 | 186 | |
| B1 | 16.3 | 7.9 | 186 | |
| B2 | 16.7 | 8.9 | 200 | |
| B3 | 17.8 | 9.3 | (1) | |
| B3-4 | 17.9 | 9.3 | (1) | 52 |
| | | 10.0 | (1) | 20 |
| | | 10.5 | (1) | 28 |
| B5 | 18.8 | 8.7 | (1) | |

| Factor | HPLC RT | GC-RT | MW | % |
|---|---|---|---|---|
| C1 | 19.6 | 10.2 | 214 | |
| C2 | 20.1 | 10.6 | 214 | |
| C3 | 20.7 | 10.0 | 214 | |
| C4 | 20.8 | 10.6 | 214 | |
| C5 | 21.0 | 11.4 | 226 | |
| C5-6 | 21.3 | 11.3 | 226 | 33 |
| | | 11.4 | 226 | 67 |

(1) GC Mass spectroscopy not done. Based on GC-RT, the molecular weight is presumed to be 200.

Absolute GC retention times, of course, will vary with conditions of a given run. In the same GC assay, a commercially available standard mix of methyl esters of $C_{8-12}$ fatty acids (Supelco) had the following GC retention times:

| | |
|---|---|
| $C_7H_{15}CO_2CH_3$ | 5.2 |
| $C_8H_{17}CO_2CH_3$ | 6.6 |
| $C_9H_{19}CO_2CH_3$ | 7.9 |
| $C_{10}H_{21}CO_2CH_3$ | 9.3 |
| $C_{11}H_{23}CO_2CH_3$ | 10.6. |

The following table shows the predicted structure (number of carbon atoms, presence of hydroxyl (if any), unsaturation (if any) and branching of the chain (if any)) of the fatty acid ($R_3COOH$) of each factor.

| Factor | Formula ($R_3COOH$) |
|---|---|
| F1 | $C_{11}$, OH |
| 2 | $C_{11}$, OH, olefin |
| 3 | $C_{11}$, OH |
| G1 | $C_{12}$, olefin |
| 2 | $C_{12}$, OH |
| 3 | $C_{12}$, OH |
| H,J,K,L1,L2 | $C_{12}$, OH |
| L3 | $C_{12}$, OH, olefin |
| M1,2 | $C_{13}$, OH |
| 3 | $C_{12}$, OH |
| N,O,P1 | $C_{13}$, OH |
| P2, | $C_{10}$, OH |
| 3 | $C_9$ |
| D | $C_{10}$, olefin |
| Q | $C_{10}$, OH |
| R | $C_{10}$, branched, olefin |
| S | $C_{10}$, branched |
| B1 | $C_{10}$ |
| B2, B4, B5 | $C_{11}$, branched |
| B3 | $C_{11}$ |
| C1, C3 | $C_{12}$, branched |
| C2, C4 | $C_{12}$ |
| C5, C6 | $C_{13}$, branched |

Each of the antibiotics of the invention was analyzed by fast atom bombardment mass spectroscopy (FAB-MS). The molecular weights (M + H) of clusters of each factor are shown in the table which follows.

| Factor | MW (M+H) |
|---|---|
| F1,2,3 | 1817 |
| G,H,J,K,L | 1831 |
| M1 | 1815 |
| 2 | 1829 |
| 3 | 1845 |
| N,O | 1845 |
| P1 | 1845 |
| 2 | 1803 |
| 3 | 1773 |
| Q | 1803 |
| D | 1785 |
| R | 1785 |
| S | 1787 |
| B1 | 1771 |
| 2 | 1801 |
| B3, B4 | 1801 |
| B5 | 1785 |
| C1 | 1815 |
| C2 | 1815 |
| C3, C4 | 1799 |
| C5, C6 | 1829 |

The mannosyl pseudoaglycones ($R_2$ = H) of the AAD 216 antibiotics have a molecular weight of 1457, except that the molecular weight of factors M2, M3, B1, B5, C3 and C4 is 1441, because these are desoxy analogs. The mannosyl pseudoaglycones of all of the factors, prepared by hydrolysis of the complex, migrated with the mannosyl pseudoaglycone of AAD 216 A, B and C in HPLC, except, of course, for the mannosyl pseudoaglycones of the desoxy analogs. For comparative purposes, the following table lists the GC-RT, the atomic masses (M+H) and the predicted empirical formulae of the fatty acids ($R_3COOH$) of AAD 216 A, B and C and the atomic masses by FAB-MS of AAD 216 A, B and C.

| Factor | MW (M+B) | GC R-T | MW | Formula ($R_3COOH$) |
|---|---|---|---|---|
| A | 1787 | 7.9 | 186 | $C_{10}$ |
| B | 1801 | 8.7 | 200 | $C_{11}$ (branched) |
| C | 1815 | 10.0 | 214 | $C_{12}$ (branched) |

Thus, the factors of this invention can be distinguished from factors A, B or C and from each other by HPLC and/or by gas chromatography of esters of $R_3COOH$. For example, factors S and A have the same molecular weight and their fatty acids have the same molecular weight. They are distinguishable, however, by the fact that the GC RT of the fatty acid ester derived from Factor A is relatively longer than that of the fatty acid ester derived from Factor S, apparently owing to the S side chain being a branched, rather than a straight chain, alkyl. Similarly, B2, B4 and B5 can be distinguished by the GC-RT of esters of $R_3COOH$, owing to $R_3$ in each being a different isomer. Also, B5 is the desoxy analog of B, and C3 is the desoxy

analog of C4. Therefore, these factors are distinguishable by HPLC of the factors or the aglycone or pseudoaglycones.

For purposes of this disclosure, factors shown to comprise multiple factors which were not further separated are treated as single factors. Thus, factors F1, F2 and F3 are considered to comprise factor F, factors G1, G2 and G3 to comprise factor G; factors L1, L2 and L3 to comprise factor L; factors M1, M2 and M3 to comprise factor M; and factors P1, P2 and P3 to comprise factor P. These complexes can be further purified into the individual components by the techniques disclosed herein.

The pseudoaglycones of the AAD 216 factors of this invention are prepared by the partial acidic hydrolysis of the AAD 216 antibiotics. The aglycone and the pseudoaglycones of the AAD 216 antibiotics are represented by the following general structural formula (I):

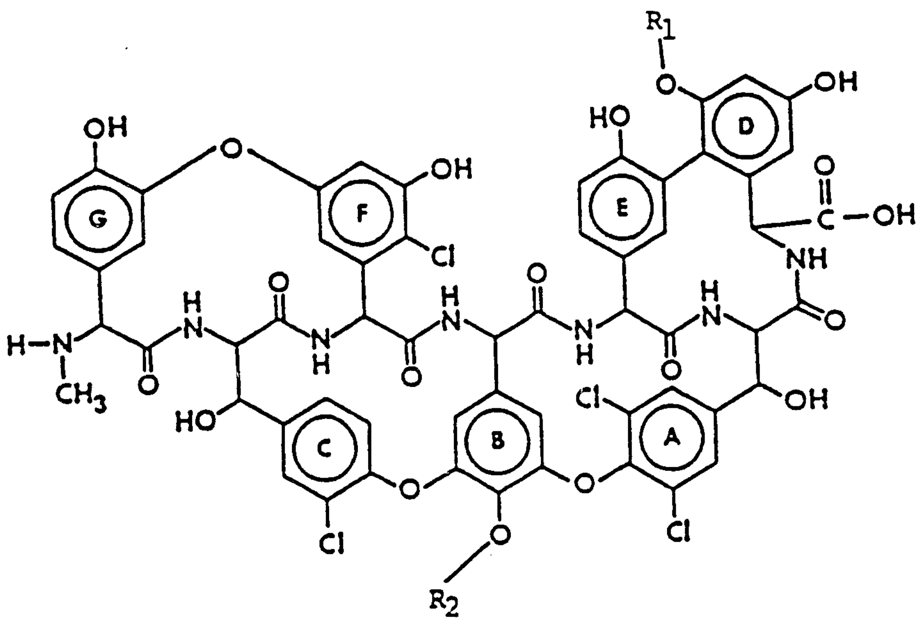

(I)

wherein $R_1$ is hydrogen or a mannosyl radical and $R_2$ is hydrogen or a glycolipid radical derived from the hydrolyzed AAD 216 antibiotic with the proviso that at least one of $R_1$ and $R_2$ is hydrogen.

The hydrolysis of the AAD 216 antibiotics proceeds along two pathways to afford the AAD 216 aglycone of the formula (I) wherein $R_1$ and $R_2$ are hydrogen as follows:

AAD 216 Antibiotics - mannose Pathway B → AAD 216 Factor Pseudoaglycones

AAD 216 Antibiotics - glycolipid Pathway A ↓ AAD 216 Mannosyl Pseudoaglycone

AAD 216 Factor Pseudoaglycones Pathway B - glycolipid ↓ AAD 216 Aglycone

AAD 216 Mannosyl Pseudoaglycone Pathway A - mannose → AAD 216 Aglycone

The initial step of the hydrolysis pathway A involves loss of the glycolipid radicals from the AAD 216 antibiotics to yield the pseudoaglycone of formula (I) wherein $R_1$ is mannosyl and $R_2$ is hydrogen. This pseudoaglycone is denominated the mannosyl pseudoaglycone. It is independent of which AAD 216 antibiotic is hydrolyzed, except that hydrolysis of a desoxy analog will yield a desoxy mannosyl pseudoaglycone. Subsequent hydrolysis of the AAD 216 pseudoagylcone yields the AAD 216 aglycone upon loss of the mannosyl radical.

The initial step of the hydrolysis pathway B involves loss of the mannosyl radical from the AAD 216 antibiotics to give pseudoaglycones of the formula (I) wherein $R_1$ is hydrogen and $R_2$ is a glycolipid radical. Accordingly, these pseudoaglycones are identified by the particular AAD 216 antibiotic hydrolyzed, e.g., the AAD 216A pseudoaglycone, the AAD 216B pseudoaglycone, the AAD 216C pseudoaglycone, and so on.

When the AAD 216 complex is hydrolyzed, a mixture of these pseudoaglycones, or "factor pseudoaglycones", will result.

The mannosyl pseudoaglycone of AAD 216 has the following characteristics:

(a) pale white-yellow solid which decomposes at 300-350 °C;

(b) an empirical formula $C_{65}H_{55}N_7O_{24}Cl_4$;

(c) an approximate elemental composition of 47.54 percent carbon, 4.00 percent hydrogen, 5.89 percent nitrogen and 8.63 percent chlorine when the water content was 12 percent;

(d) an infrared absorption spectrum in potassium bromide which exhibits peaks at the following wave numbers in $cm^{-1}$: 3400, 1660, 1610, 1590, 1510, 1460, 1430, 1390, 1300 1230, 1180, 1150, 1120, 1060, 1010, 970 and 810;

(e) a fast atom bombardment (FAB) mass spectrum with M+H at 1458 (major cluster);

(f) an ultraviolet spectrum in acetonitrile:water (1:1) which exhibits an absorption maximum at 281 nm under acid conditions with an $E_{1\%}$ = 79.7 and at 300 nm under basic conditions with an $E_{1\%}$ = 136;

(g) a carbon nuclear magnetic resonance spectrum at 90.56 MHz in $CD_3OD$:$D_2O$ (1:9) at a pH of 8.7 which exhibits the following chemical shifts in parts per million (ppm) relative to TMS as standard: 177.9, 174.6, 171.7, 170.9, 170.0, 169.2, 162.3, 158.8, 157.9, 155.2, 155.1, 153.9, 151.9, 149.7, 147.6, 147.2, 144.4, 141.0, 139.0, 138.8, 137.5, 136.1, 134.6, 130.7, 130.0, 129.8, 129.2, 128.9, 128.7, 127.5, 127.3, 126.9, 126.4, 126.0, 125.2, 122.7, 122.2, 121.1, 119.9, 119.7, 118.4, 116.6, 110.7, 110.4, 108.5, 104.4, 103.3, 100.5, 98.1, 74.0, 72.1, 71.6, 71.4, 70.8, 67.4, 65.8, 63.7, 62.2, 61.6, 60.2, 56.0, 55.9, 55.0 and 32.9; and

(h) $pK_a$ values in acetonitrile:water (3:7) as follows: 3.3, 7.1, 8.3, 9.1, 10.0 and 11.2 $pK_a$ values above 11.2 not determined.

The desoxy mannosyl pseudoaglycone lacks a benzylic hydroxyl and otherwise has substantially the same characteristics.

The AAD 216 aglycone, represented by the structural formula (1) wherein $R_1$ is -H and $R_2$ is -H has the following characteristics:

(a) pale white-yellow solid which decomposes at 300 to 350 °C;

(b) an empirical formula $C_{59}H_{45}N_7O_{19}Cl_4$;

(c) an approximate elemental composition of 47.92 percent carbon, 3.78 percent hydrogen, 6.58 percent nitrogen and 9.50 percent chlorine when the water content is 10.70 percent;

(d) an infrared absorption spectrum in potassium bromide which exhibits peaks at the following wave numbers in $cm^{-1}$: 3400, 1660, 1610, 1590, 1510, 1460, 1430, 1390, 1300, 1240, 1150, 1080, 1060 and 1010;

(e) a fast atom bombardment (FAB) mass spectrum with M+H at 1296 (major cluster);

(f) an ultraviolet spectrum in acetonitrile:water (1:1) which exhibits an absorption maximum at 281 nm under acidic conditions with an $E_{1\%}$ = 83 and at 300 nm under basic conditions with an $E_{1\%}$ = 140;

(g) a carbon magnetic resonance spectrum at 90.56 MHz in $(CD_3)_2SO$ at a pH of 3.3 which exhibits the following chemical shifts in parts per million (ppm) using TMS as the internal standard: 172.6, 172.1, 170.0, 169.2, 168.4, 167.2, 167.0, 157.2, 156.4, 155.5, 155.0, 154.6, 151.4, 147.5, 145.8, 144.7, 142.8, 141.7, 139.0, 138.7, 136.2, 135.6, 134.5, 128.5, 128.2, 128.0, 127.9, 127.8, 127.4, 127.3, 126.3, 126.0, 125.3, 125.0, 121.1, 118.1, 117.7, 117.5, 116.6, 113.7, 108.7, 106.3, 105.9, 105.4, 103.7, 102.5, 71.3, 70.1, 65.3, 61.4, 60.1, 56.8, 54.8, 53.9, 53.6 and 33.4; and

(h) $pK_a$ values in acetonitrile:water (3:7) as follows: 3.3, 7.1, 8.4, 9.2, 10.1 and 11.4 $pK_a$ values above 11.4 not determined.

The desoxy aglycone lacks a benzylic hydroxyl and otherwise has substantially the same characteristics.

The aglycones and pseudoaglycones of the instant invention are conveniently prepared from the AAD 216 antibiotics along the following lines:

The mannosyl pseudoaglycone is prepared by hydrolyzing the AAD 216 antibiotics, individually, in mixtures or in the natural complex, in aqueous organic solvent and very dilute mineral acid at reflux until a substantial amount of the AAD 216 mannosyl pseudoaglycone is formed and subsequently isolating it from the reaction mixture. Illustrative of this hydrolysis process is treating any AAD 216 antibiotic of the invention in 10 percent aqueous acetonitrile with 0.001 N hydrochloric acid at reflux for 48 hours and then isolating the mannosyl pseudoaglycone by chromatographic means.

The AAD 216 aglycone and the individual factor pseudoaglycones are prepared by hydrolysis of the appropriate factor or factors in a polar organic solvent and dilute mineral acid at elevated temperature until a substantial amount of the hydrolysis products are formed and subsequently isolating the individual desired products. Illustrative of this hydrolysis process is treating any AAD 216 antibiotic of the invention in

dimethylsulfoxide with 5 percent hydrochloric acid at 100 °C for 15 minutes and then isolating the AAD 216 aglycone by chromatographic means.

Alternatively, the AAD 216 aglycone and the mannosyl and factor pseudoaglycones are prepared by mild acid hydrolysis of the AAD 216 complex or a mixture of selected individual factors followed by a chromatographic isolation of the desired compounds.

BIOLOGICAL ACTIVITY DATA

The in vitro minimum inhibitory concentrations (MIC) of various AAD 216 factors were determined for a number of microorganisms using standard microtiter assay procedures.

The aglycone and factor pseudoaglycones (PAG) were neutralized with sodium bicarbonate prior to testing. Results (micrograms per ml) are shown in Tables A and B, which follow and in which test organisms 1-5 were various strains of Staphylococcus aureus, 6-9 and 13-16 were various strains of Staphylococcus epidermidis, 10-11 were strains of Staphylococcus faecalis and 12 was a Listeria monocytogenes. The inoculum sizes were about $10^5$ colony forming units per ml.

TABLE A

| ANTIBIOTIC | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| Vancomycin | 1.6 | 1.6 | 1.6 | 1.6 | 3.1 | 3.1 | 3.1 | 3.1 | 3.1 | 6.3 | 3.1 | 1.6 | 3.1 | 3.1 | 3.1 | 3.1 |
| Teichomycin | 1.6 | 3.1 | 0.4 | 1.6 | 3.1 | 6.3 | 12.5 | 25 | 6.3 | 0.4 | 0.2 | 0.8 | 12.5 | 3.1 | 6.3 | 6.3 |
| AAD216A | 3.1 | 3.1 | 0.8 | 0.8 | 6.3 | 25 | 50 | 100 | 50 | 0.8 | 0.8 | 0.4 | 25 | 12.5 | 12.5 | 6.3 |
| AAD216 Complex | 25 | 25 | 12.5 | 12.5 | 50 | > 50 | > 50 | > 50 | > 50 | 25 | 12.5 | 12.5 | > 50 | > 50 | > 50 | > 50 |
| AAD216F | 25 | ~ 12.5 | 6.3 | 6.3 | 50 | > 50 | > 50 | > 50 | > 50 | 6.3 | 6.3 | 12.5 | > 50 | 50 | > 50 | 50 |
| AAD216G | 12.5 | 12.5 | 3.1 | 3.1 | 25 | 50 | 100 | > 100 | 100 | 6.3 | 6.3 | 6.3 | 100 | 25 | 50 | 25 |
| AAD216H | 12.5 | 12.5 | 3.1 | 6.3 | ~ 25 | 100 | > 100 | > 100 | > 100 | 6.3 | 6.3 | ~ 3.1 | > 100 | 50 | 50 | 25 |
| AAD216J | 12.5 | 12.5 | 3.1 | 3.1 | 50 | 100 | > 100 | > 100 | > 100 | 6.3 | 12.5 | 6.3 | > 100 | 50 | 100 | 50 |
| AAD216K | 12.5 | 12.5 | 6.3 | 6.3 | 50 | 100 | > 100 | > 100 | > 100 | 6.3 | 6.3 | 3.1 | > 100 | 50 | 50 | 50 |
| AAD216L | 6.3 | 6.3 | 3.1 | 3.1 | ~ 12.5 | 100 | 100 | > 100 | > 100 | 3.1 | 3.1 | 1.6 | 100 | 25 | 50 | 25 |

**TABLE A (cont'd)**

| ANTIBIOTIC | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| AAD216M | 3.1 | 3.1 | 1.6 | 1.6 | ~ 6.3 | 25 | 50 | 100 | 50 | 3.1 | 6.3 | 1.6 | 50 | 12.5 | 12.5 | 12.5 |
| AAD216N | 12.5 | 12.5 | 3.1 | 3.1 | ~ 25 | 100 | > 100 | > 100 | > 100 | 6.3 | 6.3 | ~ 3.1 | > 100 | 50 | 100 | 50 |
| AAD216O | 12.5 | ~ 6.3 | 3.1 | 3.1 | 25 | 100 | > 100 | > 100 | > 100 | 3.1 | 3.1 | 1.6 | 100 | 25 | 50 | 25 |
| Methicillin | 1.6 | 1.6 | 1.6 | 1.6 | 50 | 3.1 | 3.1 | > 100 | 50 | 50 | 25 | 1.6 | 3.1 | 12.5 | ~ 6.3 | 12.5 |

TABLE B

| ANTIBIOTIC | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| Vancomycin | 1.6 | 1.6 | 1.6 | > 100 | 3.1 | ~ 1.6 | 3.1 | 3.1 | 3.1 | 3.1 | 3.1 | 1.6 | 3.1 | 3.1 | 3.1 | 3.1 |
| Teichomycin | 1.6 | 0.8 | ~ 0.4 | > 100 | 3.1 | 3.1 | 12.5 | 25 | 6.3 | 0.4 | 0.4 | 0.8 | 12.5 | 3.1 | 6.3 | 6.3 |
| AAD216A | 3.1 | 3.1 | 1.6 | > 100 | 12.5 | 25 | 50 | 100 | 50 | 0.8 | 0.8 | 0.8 | 25 | 12.5 | 12.5 | 6.3 |
| AAD216P | 12.5 | ~ 6.3 | 3.1 | > 100 | 25 | 100 | 100 | > 100 | 100 | 3.1 | 3.1 | 3.1 | 100 | 25 | 50 | 25 |
| AAD216Q | 6.3 | 3.1 | 3.1 | > 100 | 25 | 100 | 100 | 100 | > 100 | 3.1 | 3.1 | 3.1 | 50 | 25 | ~ 25 | 12.5 |
| AAD216R | 6.3 | 3.1 | 1.6 | > 100 | 12.5 | 50 | 50 | 100 | 50 | 1.6 | 1.6 | 1.6 | 50 | 12.5 | ~ 12.5 | 12.5 |
| AAD216S | 6.3 | 3.1 | 1.6 | > 100 | 12.5 | 50 | 50 | 100 | 100 | 1.6 | 1.6 | 1.6 | 50 | 25 | 25 | 6.3 |
| AAD216D | 3.1 | 3.1 | 1.6 | > 100 | 12.5 | 50 | 50 | 100 | 50 | 1.6 | 1.6 | 1.6 | 50 | 25 | 12.5 | 6.3 |
| AAD216B-1,2 | N.T. | N.T. | N.T. | N.T. | N.T. | N.T. | N.T. | N.T. | 50 | 0.4 | 0.8 | 0.8 | 25 | 6.3 | 12.5 | 6.3 |
| AAD216B-3 | 6.3 | 3.1 | 1.6 | > 100 | 12.5 | 50 | 50 | 100 | 100 | 0.8 | 0.8 | 1.6 | 50 | 25 | ~ 12.5 | ~ 6.3 |

**TABLE B (cont'd)**

| ANTIBIOTIC | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| AAD216B-3,4 | 12.5 | 6.3 | 3.1 | > 100 | 25 | 100 | 100 | > 100 | 100 | 1.6 | 1.6 | 3.1 | 50 | 50 | 50 | 12.5 |
| AAD216B-5 | 3.1 | 0.8 | 0.4 | 100 | 3.1 | 12.5 | 25 | 50 | 50 | 0.4 | 0.8 | 0.4 | 25 | 6.3 | ~ 6.3 | 6.3 |
| AAD216C-1 | ~ 3.1 | 3.1 | 1.6 | > 100 | 12.5 | 50 | 50 | 100 | 50 | 0.4 | 0.4 | 0.8 | 25 | 25 | 12.5 | 3.1 |
| AAD216C-2 | ~ 3.1 | 3.1 | 0.8 | 100 | 12.5 | 25 | 50 | 100 | 50 | 0.4 | 0.4 | 0.8 | 25 | 12.5 | 12.5 | 6.3 |
| AAD216C-3 | 1.6 | 1.6 | 0.8 | > 100 | 3.1 | 25 | 50 | 100 | 50 | 0.4 | 0.4 | 1.6 | 25 | 12.5 | 12.5 | 6.3 |
| AAD216C-4 | 3.1 | 3.1 | ~ 1.6 | > 50 | 12.5 | 50 | 50 | 50 | 50 | 1.6 | 1.6 | 1.6 | 50 | 25 | 25 | 12.5 |
| AAD216C-5 | 1.6 | 0.4 | 0.8 | 12.5 | 6.3 | 12.5 | 25 | 50 | 50 | 0.4 | 0.8 | 1.6 | 25 | 25 | 12.5 | 6.3 |
| AAD216C-6 | 6.3 | 0.4 | 0.4 | > 100 | 12.5 | 25 | 25 | 25 | 25 | 0.8 | 1.6 | 3.1 | 25 | 12.5 | 12.5 | 6.3 |
| AAD216A PAG | 0.4 | 0.4 | 0.4 | 50 | 0.4 | 0.8 | 0.8 | 3.1 | 1.6 | 0.8 | 1.6 | 1.6 | 1.6 | 0.8 | 0.8 | 0.8 |

The in vivo activity of the AAD 216 aglycone, AAD 216 mannosyl pseudoaglycone, AAD 216A pseudoaglycone and vancomycin, measured as $ED_{50}$, wag demonstrated against intraperitoneal infections with 46.8 $LD_{50}$'s of Staph. aureus HH 127 in mice by treatments with the antibiotics subcutaneously (s.c.), 1 and 5 hours post infection. The $ED_{50}$'s were as follows: AAD 216 aglycone, 7.6 mg/kg; AAD 216 pseudoaglycone, 7.6 mg/kg; AAD 216A pseudoaglycone, 5.0 mg./kg; vancomycin, 1.56 mg/kg. Similarly, the $ED_{50}$'s of AAD 216A pseudoaglycone, AAD 216B pseudoaglycone, AAD 216C pseudoaglycone and vancomycin,

determined by utilizing a similar protocol, were 12.5 mg/kg, 7.6 mg/kg, 10.8 mg/kg and 1.92 mg/kg, respectively.

The AAD 216 factors of the present invention thus exhibit antibacterial activity. Based on data for the aglycone, the mannosyl pseudoaglycone and the A, B and C pseudoaglycones, the pseudoaglycones of the AAD 216 factors of this invention also have antibacterial activity. The invention includes within its scope pharmaceutical compositions containing at least one of the AAD 216 factors of the invention and a pharmaceutically acceptable carrier. The compositions may also contain other active antibacterial agents. The compositions may be made up in any pharmaceutical form appropriate for the route of administration in question. Such compositions are exemplified by solid compositions for oral administration, such as tablets, capsules, pills, powders and granules; liquid compositions for oral administration such as solutions, suspensions, syrups and elixirs; and preparations for parenteral administration such as sterile solutions, suspensions or emulsions.

For use as an antibacterial agent, the compositions are administered so that the concentration of the active ingredient is greater than the minimum inhibitory concentration for the particular organism treated.

The activity of the AAD 216 aglycone, AAD 216 pseudoaglycone and AAD 216A pseudoaglycone was demonstrated in vitro against a total of 58 bovine mastitis isolates using the conventional agar dilution method to determine minimum inhibitory concentrations (MICs). The MICs for AAD 216 aglycone, the mannosyl pseudoaglycone and AAD 216A pseudoaglycone ranged from 0.5 to 128 µg/ml, 0.25 to 128 µg/ml and 0.03 to 128 µg/ml, respectively. In comparison, vancomycin has MICs for the same microorganisms ranging from 0.25 to 128 µg/ml.

Twenty-four antibiotics of the AAD 216 complex were evaluated in rumen and swine in vitro models to assess their potential as growth and feed efficiency enhancing agents in livestock. Each component was tested at two doses in one of two rumen and one of two swine in vitro incubations. AAD 216C4 was not included in the rumen in vitro model.

The results of the two rumen studies are combined in Tables I (5.0 ppm) and II (0.5 ppm). When added to the incubation at a level of 5 ppm, the antibiotics produced a desirable shift in the rumen fermentation which was similar to shifts observed with an AAD 216 complex. Each of the antibiotics enhanced propionate production (% PR). The AAD-216 complex enhanced propionate production by 128% over control incubations while the activity of the 24 antibiotics ranged from 109 to 149% of the control response. Each of the 24 antibiotics enhanced total volatile fatty acid production (TOTAL) while effectively maintaining alpha amino nitrogen (AAN) levels. The B 3-4 component showed low propionate enhancing properties. When the components were added at 0.5 ppm, they had little effect on rumen fermentation. Because the AAD 216 antibiotics increase rumen propionate, they are effective in treating and preventing ketosis and lactic acidosis. They are also therefore effective in improving feed utilization efficiency and in promoting growth in ruminants. Because the AAD 216 antibiotics increase propionate without significantly adversely affecting acetate and butyrate levels in the rumen, the antibiotics are effective in improving milk production, as measured by fat-corrected yield, in ruminants, including dairy cattle.

The results of the swine studies are combined in Tables III (16.67 ppm) and IV (1.67 ppm). As with the rumen data, the in vitro activity of the AAD 216 complex and the naturally occurring derivatives were similar when added at 16.67 ppm. The compounds spare glucose from microbial degradation by inhibiting the lactic acid-producing organisms (i.e. decreased LLAC production). Enhanced volatile fatty acid production (TOTAL) also was observed. AAD-216 antibiotics spared lysine from microbial destruction. When added to the swine incubations at a lower concentration (1.67 ppm), the AAD 216 components appeared to have different potencies against the lactic acid-producing bacteria. The lack of a lactic acid-VFA-glucose effect for antibiotics F, G, H, J, K, and L suggests they may be less potent than the AAD 216 complex and the other components. However, each of these components appears to be at least as active as avoparcin. At the 1.67 ppm concentration, the AAD 216 complex maintains a desirable profile and was more potent than avoparcin. The in vitro effects of AAD 216 antibiotics were similar to the in vitro activity of known growth promotants (i.e. bacitracin, avoparcin). Therefore, each of the AAD-216 antibiotics is an effective growth promoting agent in monogastric animals such as swine and poultry.

In the Tables, ACE means acetate; PRO means propionate; IBU means isobutyrate; TOTAL means total volatile fatty acids; % PR means the amount of propionate relative to TOTAL; NH3-N means amino nitrogen; LYS means lysine; AAN means alpha amino nitrogen; GLU means glucose; and, LLAC means lactic acid; AAD 216 complex includes factors A, B and C and the factors of this invention.

TABLE I

| ANTIBIOTIC | PPM | RUN# | ACE | PRO | IBU | BUT | IVA | VAL | TOTAL | % PR | NH3-N | LYS | AAN | GLU | LLAC |
|---|---|---|---|---|---|---|---|---|---|---|---|---|---|---|---|
| | | PERCENT OF CONTROL | | | | | | | | | | | | | |
| F | 5.00 | 1 | 95 | 121 | 130 | 89 | 143 | 108 | 102 | 119 | 117 | 0 | 120 | 0 | 78 |
| G | 5.00 | 2 | 111 | 144 | 126 | 104 | 139 | 111 | 117 | 123 | 112 | 0 | 104 | 0 | 143 |
| H | 5.00 | 1 | 104 | 136 | 119 | 91 | 128 | 116 | 110 | 124 | 116 | 0 | 118 | 0 | 81 |
| J | 5.00 | 2 | 97 | 144 | 140 | 97 | 143 | 109 | 109 | 132 | 110 | 0 | 98 | 0 | 94 |
| K | 5.00 | 1 | 106 | 131 | 75 | 90 | 122 | 122 | 109 | 121 | 114 | 0 | 116 | 0 | 95 |
| L | 5.00 | 2 | 97 | 136 | 153 | 100 | 141 | 111 | 108 | 125 | 113 | 0 | 103 | 0 | 118 |
| M | 5.00 | 1 | 94 | 130 | 114 | 84 | 124 | 109 | 102 | 127 | 110 | 0 | 127 | 0 | 103 |
| N | 5.00 | 1 | 106 | 143 | 116 | 90 | 125 | 112 | 112 | 128 | 120 | 0 | 125 | 0 | 80 |
| O | 5.00 | 2 | 111 | 160 | 100 | 107 | 138 | 113 | 121 | 132 | 108 | 0 | 99 | 0 | 129 |
| P | 5.00 | 1 | 94 | 131 | 135 | 85 | 123 | 102 | 103 | 128 | 116 | 0 | 126 | 0 | 86 |
| Q | 5.00 | 2 | 97 | 172 | 178 | 103 | 135 | 107 | 117 | 147 | 107 | 0 | 97 | 0 | 86 |
| R | 5.00 | 2 | 90 | 157 | 159 | 96 | 138 | 94 | 108 | 146 | 109 | 0 | 117 | 0 | 116 |
| S | 5.00 | 2 | 107 | 173 | 127 | 104 | 134 | 104 | 122 | 143 | 109 | 0 | 106 | 0 | 134 |
| D | 5.00 | 1 | 95 | 139 | 93 | 81 | 91 | 105 | 103 | 136 | 118 | 0 | 126 | 0 | 97 |
| B 1-2 | 5.00 | 2 | 94 | 172 | 127 | 108 | 136 | 94 | 115 | 149 | 110 | 0 | 108 | 0 | 152 |
| B 3 | 5.00 | 2 | 93 | 144 | 184 | 96 | 145 | 103 | 107 | 134 | 113 | 0 | 101 | 0 | 126 |
| B 3-4 | 5.00 | 1 | 113 | 122 | 93 | 103 | 126 | 114 | 113 | 109 | 108 | 0 | 107 | 0 | 88 |
| B 5 | 5.00 | 1 | 91 | 138 | 95 | 85 | 97 | 105 | 102 | 135 | 121 | 0 | 139 | 0 | 98 |
| C 1 | 5.00 | 1 | 99 | 148 | 138 | 93 | 145 | 104 | 112 | 132 | 117 | 0 | 134 | 0 | 88 |
| C 2 | 5.00 | 2 | 99 | 173 | 154 | 106 | 150 | 98 | 118 | 146 | 113 | 0 | 111 | 0 | 125 |

TABLE I (cont'd)

| ANTIBIOTIC | PPM | RUN# | ACE | PRO | IBU | BUT | IVA | VAL | TOTAL | % PR | NH3-N | LYS | AAN | GLU | LLAC |
|---|---|---|---|---|---|---|---|---|---|---|---|---|---|---|---|
| | | | PERCENT OF CONTROL | | | | | | | | | | | | |
| C 3 | 5.00 | 2 | 110 | 175 | 111 | 106 | 136 | 101 | 123 | 142 | 110 | 0 | 107 | 0 | 102 |
| C 5 | 5.00 | 1 | 99 | 143 | 129 | 99 | 154 | 112 | 113 | 127 | 118 | 0 | 127 | 0 | 73 |
| C 6 | 5.00 | 2 | 94 | 132 | 92 | 95 | 130 | 102 | 104 | 128 | 108 | 0 | 95 | 0 | 109 |
| AAD-216 Complex | 5.00 | 1 | 96 | 132 | 100 | 86 | 115 | 115 | 104 | 127 | 119 | 0 | 130 | 0 | 73 |
| AAD-216 Complex | 5.00 | 2 | 108 | 151 | 117 | 99 | 148 | 107 | 116 | 130 | 110 | 0 | 105 | 0 | 156 |
| Avoparcin | 5.00 | 1 | 91 | 116 | 115 | 85 | 111 | 93 | 97 | 119 | 117 | 0 | 120 | 0 | 101 |
| Avoparcin | 5.00 | 2 | 113 | 138 | 111 | 103 | 130 | 103 | 116 | 119 | 107 | 0 | 101 | 0 | 150 |
| Monensin | 5.00 | 1 | 101 | 136 | 113 | 64 | 170 | 113 | 102 | 133 | 119 | 0 | 194 | 0 | 449 |
| Monensin | 5.00 | 2 | 84 | 162 | 81 | 96 | 82 | 73 | 104 | 157 | 109 | 0 | 163 | 0 | 200 |

TABLE II

| ANTIBIOTIC | PPM | RUN# | ACE | PRO | IBU | BUT | IVA | VAL | TOTAL | % PR | NH3-N | LYS | AAN | GLU | LLAC |
|---|---|---|---|---|---|---|---|---|---|---|---|---|---|---|---|
| | | | PERCENT OF CONTROL | | | | | | | | | | | | |
| F | 0.50 | 1 | 105 | 106 | 115 | 104 | 116 | 102 | 105 | 101 | 102 | 0 | 112 | 0 | 83 |
| G | 0.50 | 2 | 88 | 82 | 106 | 84 | 86 | 88 | 86 | 96 | 110 | 0 | 99 | 0 | 131 |
| H | 0.50 | 1 | 99 | 99 | 103 | 100 | 104 | 99 | 99 | 100 | 102 | 0 | 103 | 0 | 104 |
| J | 0.50 | 2 | 99 | 101 | 103 | 105 | 111 | 107 | 101 | 99 | 99 | 0 | 87 | 0 | 85 |
| K | 0.50 | 1 | 96 | 102 | 135 | 103 | 126 | 101 | 101 | 101 | 101 | 0 | 106 | 0 | 87 |
| L | 0.50 | 2 | 107 | 113 | 115 | 109 | 124 | 113 | 109 | 103 | 99 | 0 | 81 | 0 | 119 |
| M | 0.50 | 1 | 107 | 106 | 91 | 101 | 104 | 104 | 105 | 101 | 96 | 0 | 108 | 0 | 108 |
| N | 0.50 | 1 | 111 | 105 | 72 | 102 | 84 | 105 | 106 | 100 | 102 | 0 | 110 | 0 | 72 |
| O | 0.50 | 2 | 103 | 117 | 129 | 106 | 130 | 111 | 108 | 108 | 99 | 0 | 96 | 0 | 126 |
| P | 0.50 | 1 | 94 | 96 | 87 | 89 | 75 | 94 | 93 | 103 | 99 | 0 | 107 | 0 | 84 |
| Q | 0.50 | 2 | 104 | 115 | 104 | 109 | 120 | 112 | 108 | 107 | 98 | 0 | 91 | 0 | 92 |
| R | 0.50 | 2 | 107 | 117 | 114 | 106 | 120 | 111 | 109 | 107 | 99 | 0 | 95 | 0 | 118 |
| S | 0.50 | 2 | 106 | 112 | 109 | 106 | 123 | 109 | 108 | 104 | 102 | 0 | 96 | 0 | 93 |
| D | 0.50 | 1 | 95 | 101 | 118 | 98 | 117 | 95 | 98 | 102 | 102 | 0 | 107 | 0 | 90 |
| B 1-2 | 0.50 | 2 | 111 | 111 | 104 | 107 | 120 | 111 | 110 | 101 | 105 | 0 | 90 | 0 | 121 |
| B 3 | 0.50 | 2 | 116 | 116 | 106 | 109 | 125 | 114 | 114 | 101 | 102 | 0 | 89 | 0 | 112 |
| B 3-4 | 0.50 | 1 | 107 | 106 | 102 | 103 | 105 | 108 | 106 | 101 | 102 | 0 | 109 | 0 | 98 |
| B 5 | 0.50 | 1 | 105 | 105 | 75 | 101 | 95 | 108 | 103 | 102 | 103 | 0 | 116 | 0 | 84 |
| C 1 | 0.50 | 1 | 106 | 104 | 85 | 100 | 96 | 110 | 103 | 101 | 102 | 0 | 107 | 0 | 101 |
| C 2 | 0.50 | 2 | 110 | 110 | 111 | 105 | 115 | 112 | 109 | 101 | 102 | 0 | 97 | 0 | 133 |

**TABLE II (cont'd)**

| ANTIBIOTIC | PPM | RUN# | ACE | PRO | IBU | BUT | IVA | VAL | TOTAL | % PR | NH3-N | LYS | AAN | GLU | LLAC |
|---|---|---|---|---|---|---|---|---|---|---|---|---|---|---|---|
| | | | -----PERCENT OF CONTROL----- | | | | | | | | | | | | |
| C 3 | 0.50 | 2 | 93 | 107 | 105 | 95 | 108 | 101 | 97 | 110 | 100 | 0 | 95 | 0 | 93 |
| C 5 | 0.50 | 1 | 100 | 105 | 103 | 105 | 114 | 112 | 104 | 101 | 104 | 0 | 106 | 0 | 73 |
| C 6 | 0.50 | 2 | 111 | 116 | 90 | 113 | 112 | 116 | 113 | 103 | 98 | 0 | 88 | 0 | 70 |
| AAD-216 Complex | 0.50 | 1 | 97 | 99 | 81 | 99 | 81 | 102 | 98 | 101 | 103 | 0 | 108 | 0 | 86 |
| AAD-216 Complex | 0.50 | 2 | 110 | 119 | 117 | 112 | 131 | 114 | 113 | 105 | 100 | 0 | 91 | 0 | 170 |
| Avoparcin | 0.50 | 1 | 103 | 101 | 96 | 104 | 104 | 98 | 102 | 99 | 102 | 0 | 110 | 0 | 100 |
| Avoparcin | 0.50 | 2 | 96 | 105 | 109 | 98 | 89 | 94 | 99 | 107 | 101 | 0 | 92 | 0 | 144 |
| Monensin | 0.50 | 1 | 110 | 96 | 90 | 98 | 195 | 122 | 105 | 91 | 112 | 0 | 131 | 0 | 95 |
| Monensin | 0.50 | 2 | 83 | 128 | 106 | 121 | 116 | 101 | 103 | 124 | 105 | 0 | 129 | 0 | 116 |

## TABLE III

| ANTIBIOTIC | PPM | RUN# | ACE | PRO | IBU | BUT | IVA | VAL | TOTAL | % PR | NH3-N | LYS | AAN | GLU | LLAC |
|---|---|---|---|---|---|---|---|---|---|---|---|---|---|---|---|
| | | PERCENT OF CONTROL | | | | | | | | | | | | | |
| F | 16.67 | 1 | 136 | 170 | 0 | 133 | 107 | 141 | 152 | 112 | 213 | 108 | 0 | 191 | 10 |
| G | 16.67 | 2 | 114 | 155 | 0 | 117 | 0 | 0 | 135 | 115 | 125 | 111 | 0 | 256 | 5 |
| H | 16.67 | 1 | 132 | 155 | 0 | 108 | 62 | 111 | 141 | 110 | 413 | 119 | 0 | 186 | 7 |
| J | 16.67 | 2 | 114 | 154 | 0 | 114 | 0 | 0 | 133 | 116 | 145 | 108 | 0 | 269 | 4 |
| K | 16.67 | 1 | 125 | 145 | 0 | 100 | 0 | 100 | 132 | 110 | 385 | 121 | 0 | 187 | 6 |
| L | 16.67 | 2 | 119 | 146 | 0 | 94 | 0 | 0 | 130 | 112 | 237 | 124 | 0 | 262 | 6 |
| M | 16.67 | 1 | 129 | 142 | 0 | 94 | 56 | 96 | 132 | 108 | 582 | 123 | 0 | 187 | 4 |
| N | 16.67 | 1 | 130 | 155 | 0 | 113 | 139 | 114 | 141 | 110 | 254 | 119 | 0 | 186 | 6 |
| O | 16.67 | 2 | 117 | 154 | 0 | 114 | 0 | 0 | 135 | 114 | 268 | 129 | 0 | 273 | 4 |
| P | 16.67 | 1 | 125 | 145 | 0 | 101 | 79 | 99 | 133 | 110 | 320 | 131 | 0 | 187 | 2 |
| Q | 16.67 | 2 | 108 | 142 | 0 | 100 | 0 | 0 | 124 | 115 | 260 | 107 | 0 | 268 | 3 |
| R | 16.67 | 2 | 108 | 143 | 0 | 104 | 0 | 0 | 124 | 115 | 246 | 130 | 0 | 277 | 4 |
| S | 16.67 | 2 | 118 | 148 | 0 | 106 | 0 | 0 | 131 | 113 | 234 | 130 | 0 | 267 | 5 |
| D | 16.67 | 1 | 126 | 145 | 109 | 96 | 0 | 100 | 132 | 109 | 432 | 129 | 0 | 187 | 3 |
| B 1-2 | 16.67 | 2 | 117 | 156 | 0 | 129 | 0 | 0 | 137 | 114 | 196 | 116 | 0 | 268 | 4 |
| B 3 | 16.67 | 2 | 111 | 145 | 0 | 118 | 0 | 0 | 128 | 113 | 235 | 116 | 0 | 280 | 5 |
| B 3-4 | 16.67 | 1 | 124 | 142 | 126 | 101 | 119 | 102 | 131 | 108 | 373 | 115 | 0 | 186 | 4 |
| B 5 | 16.67 | 1 | 135 | 148 | 0 | 104 | 0 | 110 | 138 | 107 | 489 | 121 | 0 | 196 | 4 |
| C 1 | 16.67 | 1 | 131 | 152 | 0 | 112 | 119 | 112 | 140 | 109 | 329 | 122 | 0 | 186 | 4 |
| C 2 | 16.67 | 2 | 116 | 151 | 0 | 113 | 0 | 0 | 134 | 113 | 240 | 128 | 0 | 278 | 4 |

TABLE III (cont'd)

| ANTIBIOTIC | PPM | RUN# | ACE | PRO | IBU | BUT | IVA | VAL | TOTAL | % PR | NH3-N | LYS | AAN | GLU | LLAC |
|---|---|---|---|---|---|---|---|---|---|---|---|---|---|---|---|
| | | PERCENT OF CONTROL | | | | | | | | | | | | | |
| C 3 | 16.67 | 2 | 105 | 139 | 0 | 107 | 0 | 0 | 121 | 115 | 155 | 119 | 0 | 281 | 3 |
| C 4 | 16.67 | 1 | 126 | 142 | 0 | 104 | 102 | 112 | 132 | 108 | 356 | 112 | 0 | 195 | 4 |
| C 5 | 16.67 | 1 | 135 | 152 | 418 | 107 | 34 | 110 | 142 | 107 | 413 | 117 | 0 | 188 | 5 |
| C 6 | 16.67 | 2 | 117 | 143 | 0 | 100 | 0 | 0 | 128 | 111 | 160 | 145 | 0 | 280 | 2 |
| AAD-216 Complex | 16.67 | 1 | 132 | 144 | 0 | 102 | 0 | 109 | 135 | 107 | 269 | 123 | 0 | 185 | 5 |
| AAD-216 Complex | 16.67 | 2 | 107 | 139 | 0 | 113 | 0 | 0 | 123 | 113 | 247 | 110 | 0 | 275 | 4 |
| Avoparcin | 16.67 | 1 | 128 | 147 | 441 | 102 | 0 | 106 | 136 | 108 | 482 | 118 | 0 | 187 | 10 |
| Avoparcin | 16.67 | 2 | 113 | 149 | 0 | 108 | 0 | 0 | 130 | 115 | 216 | 117 | 0 | 255 | 8 |
| Monensin | 16.67 | 1 | 49 | 38 | 0 | 89 | 118 | 115 | 47 | 81 | 21 | 151 | 0 | 235 | 16 |
| Monensin | 16.67 | 2 | 40 | 29 | 0 | 51 | 0 | 0 | 35 | 81 | 116 | 157 | 0 | 323 | 9 |

TABLE IV

| ANTIBIOTIC | PPM | RUN# | ACE | PRO | IBU | BUT | IVA | VAL | TOTAL | % PR | NH3-N | LYS | AAN | GLU | LLAC |
|---|---|---|---|---|---|---|---|---|---|---|---|---|---|---|---|
| | | PERCENT OF CONTROL | | | | | | | | | | | | | |
| F | 1.67 | 1 | 98 | 103 | 0 | 100 | 76 | 104 | 101 | 102 | 40 | 96 | 0 | 102 | 98 |
| G | 1.67 | 2 | 107 | 113 | 0 | 101 | 0 | 0 | 110 | 103 | 126 | 112 | 0 | 108 | 96 |
| H | 1.67 | 1 | 106 | 117 | 0 | 96 | 0 | 110 | 110 | 106 | 140 | 105 | 0 | 113 | 91 |
| J | 1.67 | 2 | 105 | 121 | 0 | 99 | 0 | 0 | 113 | 108 | 114 | 89 | 0 | 120 | 84 |
| K | 1.67 | 1 | 105 | 112 | 0 | 95 | 0 | 102 | 107 | 105 | 229 | 101 | 0 | 106 | 91 |
| L | 1.67 | 2 | 112 | 122 | 0 | 100 | 0 | 0 | 116 | 105 | 185 | 108 | 0 | 115 | 91 |
| M | 1.67 | 1 | 131 | 143 | 54 | 86 | 102 | 90 | 133 | 108 | 307 | 109 | 0 | 158 | 41 |
| N | 1.67 | 1 | 124 | 144 | 0 | 117 | 125 | 124 | 133 | 108 | 115 | 105 | 0 | 141 | 59 |
| O | 1.67 | 2 | 121 | 150 | 0 | 113 | 0 | 0 | 135 | 111 | 193 | 112 | 0 | 170 | 56 |
| P | 1.67 | 1 | 137 | 151 | 0 | 97 | 0 | 102 | 140 | 108 | 303 | 125 | 0 | 167 | 28 |
| Q | 1.67 | 2 | 113 | 138 | 0 | 81 | 0 | 0 | 123 | 112 | 239 | 113 | 0 | 219 | 23 |
| R | 1.67 | 2 | 115 | 150 | 0 | 100 | 0 | 0 | 131 | 115 | 202 | 120 | 0 | 254 | 10 |
| S | 1.67 | 2 | 131 | 163 | 0 | 108 | 0 | 0 | 145 | 113 | 281 | 127 | 0 | 241 | 10 |
| D | 1.67 | 1 | 136 | 153 | 0 | 104 | 129 | 100 | 141 | 108 | 274 | 120 | 0 | 187 | 9 |
| B 1-2 | 1.67 | 2 | 123 | 164 | 0 | 118 | 0 | 0 | 142 | 115 | 262 | 110 | 0 | 248 | 9 |
| B 3 | 1.67 | 2 | 121 | 152 | 0 | 108 | 0 | 0 | 135 | 112 | 126 | 118 | 0 | 239 | 14 |
| B 3-4 | 1.67 | 1 | 133 | 143 | 0 | 123 | 85 | 114 | 137 | 105 | 151 | 104 | 0 | 130 | 64 |
| B 5 | 1.67 | 1 | 140 | 153 | 191 | 100 | 62 | 101 | 143 | 107 | 455 | 114 | 0 | 185 | 9 |
| C 1 | 1.67 | 1 | 125 | 142 | 0 | 95 | 0 | 92 | 130 | 109 | 511 | 118 | 0 | 186 | 9 |
| C 2 | 1.67 | 2 | 125 | 162 | 0 | 113 | 0 | 0 | 142 | 115 | 138 | 119 | 0 | 232 | 11 |

TABLE IV (cont'd)

| ANTIBIOTIC | PPM | RUN# | ACE | PRO | IBU | BUT | IVA | VAL | TOTAL | % PR | NH3-N | LYS | AAN | GLU | LLAC |
|---|---|---|---|---|---|---|---|---|---|---|---|---|---|---|---|
| | | | PERCENT OF CONTROL | | | | | | | | | | | | |
| C 3 | 1.67 | 2 | 120 | 152 | 0 | 103 | 0 | 0 | 134 | 113 | 255 | 107 | 0 | 255 | 9 |
| C 4 | 1.67 | 1 | 129 | 135 | 0 | 119 | 119 | 107 | 131 | 103 | 252 | 101 | 0 | 153 | 49 |
| C 5 | 1.67 | 1 | 143 | 154 | 0 | 114 | 159 | 104 | 145 | 106 | 321 | 111 | 0 | 172 | 28 |
| C 6 | 1.67 | 2 | 113 | 125 | 0 | 110 | 0 | 0 | 118 | 106 | 118 | 106 | 0 | 132 | 77 |
| AAD-216 Complex | 1.67 | 1 | 144 | 156 | 577 | 118 | 62 | 93 | 150 | 105 | 584 | 111 | 0 | 185 | 13 |
| AAD-216 Complex | 1.67 | 2 | 124 | 159 | 0 | 113 | 0 | 0 | 140 | 113 | 184 | 111 | 0 | 240 | 15 |
| Avoparcin | 1.67 | 1 | 96 | 99 | 54 | 82 | 91 | 92 | 96 | 103 | 197 | 93 | 0 | 102 | 104 |
| Avoparcin | 1.67 | 2 | 97 | 109 | 0 | 87 | 0 | 0 | 102 | 107 | 31 | 107 | 0 | 92 | 109 |
| Monensin | 1.67 | 1 | 123 | 138 | 0 | 114 | 82 | 124 | 130 | 107 | 165 | 118 | 0 | 196 | 6 |
| Monensin | 1.67 | 2 | 104 | 127 | 0 | 102 | 0 | 0 | 116 | 110 | 152 | 122 | 0 | 277 | 5 |

The feed compositions of this invention comprise the normal feed rations of the meat and milk producing animals supplemented by a quantity of an active ingredient selected from one or more of the AAD 216 factors of the invention which is effective for improving the growth rate and feed efficiency of the animals but which is not toxic or noxious to a degree that the animals will reduce ingestion of the ration. The quantity of the active ingredient will vary, as is known to the art, with factors such as the cost of the

ingredient, the species and the size of the animals, the relative activity of the compound of formula I and the type of feed ration used as the basal feed. The quantity of the active ingredient to increase propionate, to improve feed utilization efficiency, to promote growth and to treat or prevent ketosis or lactic acidosis should be an amount which increases propionate levels in the rumen or cecum and, to improve milk production, the quantity should be an amount which increases propionate level in the rumen but which does not significantly decrease acetate and butyrate levels. Such quantities are readily determined by standard techniques. See, for example, Scheifinger, U.S. Patent 4,430,328 and Smith et al., GB-2,137,087-A, with respect to milk

production. Representative feed rations for swine and poultry are as follows:

A swine ration for growing hogs of 40-100 pounds body weight is prepared using the following formula:

| | |
|---|---|
| Corn, ground | 78.15% |
| Soybean oil meal, 44% | 17.0% |
| Meat scraps, 50% | 3.0% |
| Oyster shell flavor | 0.4% |
| Bone meal | 0.5% |
| Zinc oxide | 0.01% |
| Vitamin A, B, $B_{12}$ & D supplement | optional |

A chicken ration for broilers is prepared using the following formula:

| | |
|---|---|
| Yellow corn meal | 67.35% |
| Soybean oil meal | 24.00% |
| Menhaden fish meal | 6.00% |
| Steamed bone meal | 1.00% |
| Ground limestone | 1.00% |
| Iodized salt | 0.34% |
| 25% choline chloride | 0.13% |
| Vitamin $B_{12}$ | 0.10% |
| Manganese sulfate | 0.02% |
| Vitamin mix | 0.06% |

Swine feed from weanling to fattening or finishing rations may be supplemented. Swine eat from about 2 lb. of ration per day (for a 25 lb. pig) to 9 lb. per day (for a 150 lb. pig). Most rations are comprised of a corn base supplemented with legume silage, wheat bran, oats; barley, molasses or a protein supplement.

Poultry feeds comprise starter rations, broiler rations and laying rations. The rations are usually based on ground corn, corn meal or soybean meal. The broiler rations, often, contain high energy supplements such as added fats, proteins and vitamins. Turkey rations are similar, but comprise only a starting ration and a growing ration. Chickens or pheasants eat from .03-0.3 lbs. of feed per day, turkeys twice that much. Estimated intake of feed is dependent on the weight and age of the meat producing animal.

The active ingredients selected from the AAD 216 antibiotics or a mixture thereof are mixed uniformly with such feed rations to give supplemented rations which are, then fed as to custom, which is, most often, ad libitum. Conveniently, to do this, a premix of the supplemental growth promotant of this invention, optionally combined with or without other supplements known to this art such as an anthelmintic, a nitrogen source or an antibiotic, for example, virginiamycin or oxytetracycline is prepared by the manufacturer for sale to the formulators or feed lot operators. The concentration of the active ingredients in the premix is usually from 5-75% by weight or a concentration 100-2000 times greater than that in the complete feed ration. The premix form may be liquid or solid. Premix vehicles are corn oil, cottonseed oil, molasses or distillers solubles to form a liquid premix preparation. Sucrose, lactose, corn meal, ground corn, flour, calcium carbonate or soybean meal are often used as bases for solid premix preparations. The premix composition is, then, mixed uniformly with whole ration which is fed to the target animal. Such premix compositions are included in the term "feed compositions" as used herein.

The concentration of the active ingredients in the complete ration is a nontoxic but active quantity chosen, for example, from a range of about 1-1000 parts of active ingredient by weight per million parts of whole feed (ppm) or about 2-115 grams per ton. Advantageously, a nontoxic quantity of active ingredient is chosen from the range of 10-50 ppm.

The method of this invention comprises feeding to monogastric or ruminant, meat or milk producing animals, especially beef and dairy cattle, sheep, swine and poultry, an effective growth promoting but nontoxic quantity of an AAD 216 antibiotic. Other monogastric animals whose digestive tract also features fermentation in a cecum or cecum-like chamber are rabbits and horses.

The supplemented feed rations, described above, are presented to the animal by methods known to the art. Ad libitum feeding in the pasture, pen or growing shed is most convenient to increase the growth and milking rate of the animal and to increase the feed efficiency of the operation.

The following examples are illustrative of the production, isolation and purification of the antibiotics of the present invention and are not therefore to be considered in limiting the present invention as described in the claims appended hereto.

EXAMPLE 1

First Seed Stage

The entire growth of a slant of K. aridum was suspended in 10 ml of sterile water and aseptically inoculated into a 4 l. aspirator bottle containing 500 ml of medium 13 H. After incubation on a rotatory shaker, 250 rpm, at 28°C for 3 days, the mature culture was then employed to inoculate the 14 l. glass vessel fermentor of Example 2 to produce a second seed stage.

EXAMPLE 2

Second Seed Stage

A 14 l. glass vessel fermentor (New Brunswick Model 19) with 10 l. of sterile medium 13H was aseptically inoculated with 500 ml of vegetative culture from Example 1. The vessel was operated according to the following schedule:

Agitation: 400 rpm from 0 - 72 hr.
Aeration: 0.4 v/v/m* from 0 - 72 hr.
Temp.: 28°C.

Five liters of the resultant vegetative culture was then employed to inoculate the 75 l. fermentor of Example 3 to produce a third seed stage.

EXAMPLE 3

Third Seed Stage

A 75 l. fermentator (Chemapec) was run in similar fashion to Example 2. Five liters of the vegetative culture from Example 2 was used to inoculate the 75 l. fermentor containing 50 l. of medium 13H. The fermentation apparatus was operated as follows:

Agitation: 250 rpm from 0 - 72 hr.
Aeration: 0.4 v/v/m from 0 - 72 hr.
Temp.: 28°C.

Fifty liters of the resultant vegetative culture was then employed to inoculate the 750 l. fermentor of Example 4 to produce the desired AAD 216 complex.

EXAMPLE 4

Production of AAD 216 Complex

A 750 l. fermentor (ABEC) was operated in a similar fashion to Example 3. Fifty liters of the vegetative culture of Example 3 was used to aseptically inoculate the 750 l. fermentor containing 600 l. of medium V-2.

*v/v/m - vol. of air per vol. of medium per minute

The fermentor was operated as follows:

Agitation: 120 rpm from 0 - 168 hr.
Aeration: 0.3 v/v/m from 0 - 168 hr.
Temp.: 26° C.

The fermentation broth contained the AAD 216 complex which was composed of the individual factor antibiotics as follows:

AAD 216A = 50 µg/ml;
AAD 216B = 60.8 µg/ml; and
AAD 216C = 43.5 µg/ml,

as well as lesser amounts of the AAD 216 mannosyl pseudoaglycone and the AAD 216 factors of this invention.

EXAMPLE 5

Production of AAD 216 Complex

A 450 l. fermentor (Chemapec) was operated in a similar fashion to Example 4. Thirty liters of vegetative culture (third seed stage) produced according to Example 3 was used to aseptically inoculate the 450 l. fermentor containing 300 l. of medium V-2. The fermentor was oeprated as follows:

Agitation: 120 rpm from 0 - 168 hr.
Aeration: 0.4-0.5 v/v/m
Temperature: 26° C.

The fermentation broth contained the AAD 216 complex which was composed of the individual factor antibiotics as follows:

AAD 216A = 46.2 µg/ml
AAD 216B = 75 µg/ml
AAD 216C = 48 µg/ml,

as well as lesser amounts of the AAD 216 mannosyl pseudoaglycone and the AAD 216 factors of this invention.

EXAMPLE 6

Isolation of AAD 216 Complex

Whole fermentation broth (600 l.) from Example 4 was clarified by rotary drum filtration (Komline-Sanderson, Laboratory Scale Model) using filter aid (Hyflo Supercel, Johns-Manville Products Corp.) at existing broth pH (pH 7.7-8.2). The broth filtrate (420 l.) was chilled to 4° C by batch treatment of 100 L. volumes in a vat placed in a cold bath (methanol/dry ice). The chilled broth filtrate was then precipitated by slow addition of concentrated hydrochloric acid, with mixing, to pH 3.0. The resulting precipitate was recovered by rotary vacuum filtration using filter aid as previously described. The filter aid-product precipitate cake was extracted by mixing with deionized water (55 l.) and adjusting to pH 7.0 for 10 minutes. The aqueous extract thus obtained was filtered through Whatman #4 filter paper to remove the filter aid. The filtrate so obtained was applied to two XAD-7 resin columns (8.5 x 110 cm.) at a flow rate of 0.5 vols./hr. After washing with 8 volumes of deionized water (pH 7.0) the desired AAD 216 complex was recovered by elution with aqueous acetonitrite (50-100%). The eluate(s) containing the desired AAD 216 complex was concentrated by evaporation using a rising film evaporator at 35° C. The resulting aqueous concentrate was freeze-dried on a Virtis shelf lyophilizer, yielding 85.1 g of AAD 216 complex, which comprised a mixture of AAD 216 factors of the invention, the AAD 216 mannosyl pseudoaglycone and, as major factors, AAD 216A, B and C.

EXAMPLE 7

Isolation of AAD 216A, AAD 216B and AAD 216C

A sample of an AAD 216 complex isolated according to Example 6 (25 g.), was dissolved in one liter of 17% acetonitrile in 0.1 M pH 6 phosphate buffer and the pH was adjusted to 6.3. This solution was loaded onto a Waters Prep 500® HPLC with a column of Whatman Partisil® 40 (ODS-3) (50 cm. x 4.8 cm.) at a flow rate of 200 ml/minute. The column was then eluted using the following gradient:

(1) 20% aqueous acetonitrile and buffer solution until polar material (ultraviolet detector at 210 nm) was eluted;

(2) 22% acetonitrile buffer solution until AAD 216A was eluted;

(3) 24% acetonitrile buffer solution until AAD 216B was eluted;

(4) 26% acetonitrile buffer solution until AAD 216C was eluted; and

(5) 50% aqueous acetonitrile.

The appropriate fractions which were eluted from the HPLC column were then desalted as described below to afford the pure individual antibiotics: AAD 216A, 2.0 g; AAD 216B, 1.7 g; and AAD 216C, 1.3 g.

The desalting procedure involved a pooling of the appropriate fractions from the HPLC and removal of the acetonitrile at reduced pressure. The resulting aqueous samples were loaded onto an XAD-7 resin column and eluted with deionized water until the conductivity of the outflow was less than 25 micro MHO. The column was then eluted with aqueous acetonitrile (40-60%) and the eluant lyophilized to afford the desired products.

EXAMPLE 8

Isolation of AAD 216 Antibiotics

A sample of a complex isolated substantially by the procedure of Example 6 (about 60 g, containing about 15 g of complex) was adjusted to pH 7.0 and passed through an affinity column of Affi-Gel® 10-D-ala-D-ala. The gel was washed with 2 L of 0.02 M phosphate buffer (pH6), 2 L of water and 1 L of 10% acetonitrile-water and eluted with 4 L of 50% acetonitrile-1N $NH_4OH$. The ammonia was removed by concentration in vacuo and the aqueous solution was lyophilized to yield 13 g of pure AAD 216 complex, comprising the AAD 216 factors of the invention, the AAD 216 mannosyl pseudoaglycone and, as major components, AAD 216A, B and C.

A 1 mg/L solution of this complex was assayed by HPLC on a Beckman Ultrasphere ODS column, 4.6 x 250 mm, using a gradient of 25 to 40% acetonitrile in 0.1 M phosphoric acid, pH 3.2, to produce the chromatogram which is reproduced herein as the Figure.

EXAMPLE 9

Isolation of AAD 216 Antibiotic Factors

The individual AAD 216 factors, including factors A, B and C, were purified for further analysis by preparative HPLC on a column of Whatman Partisil ODS-3, 10 microns, using various concentrations of acetonitrile in 0.1 M phosphate buffer (pH 6) at 10 ml/min. Whatman Partisil is a silica adsorbent. The AAD 216 factors of the invention were eluted as follows:

(1) 20-21% acetonitrile/buffer until Factors F-O were eluted;

(2) 26% acetonitrile/buffer until Factors P-S were eluted;

(3) 28% acetonitrile/buffer until Factor D was eluted;

(4) 30% acetonitrile/buffer until Factors B1-5 were eluted; and

(5) 32% acetonitrile until Factors C1-6 were eluted.

Fractions containing purified components were isolated substantially as described in Example 7 and lyophilized to a white powder.

All products were found to contain mannose, to have a typical combustion analysis of 45-50% carbon, 4.5% hydrogen, 5-6% nitrogen, 6-8% chlorine, 10% water by Thermalgravimetric analysis. All components had a UV absorption maximum at 280 nm with $E_{1\%}$ values varying from 40 to 55 depending on purity. All components had similar IR spectra (KBr) of about 3400, 1660, 1610, 1590, 1510, 1470, 1440, 1410, 1390, 1320, 1300, 1240, 1120, 1060, 1020 and 950 $cm^{-1}$.

EXAMPLE 10

Alternate Isolation of AAD 216 Antibiotic

A mixture of components F to S was isolated from the lyophilized 20% acetonitile cut (Fraction #1 of Example 7) using affigel-D-ala-D-ala substantially by the procedure described in Example 8.

The product was fractionated on the Whatman Magnum 20 column as described in Example 8. Components D, B-1 to B-6 and C1 to C6 were isolated in an analogous manner from side cuts of fractions

2-4 of Example 7.

EXAMPLE 11

Preparation of AAD 216 Mannosyl Pseudoaglycone

AAD 216A (1.2 g) was partially dissolved in 10 percent aqueous acetonitrile and 0.001 N hydrochloric acid (total volume 1500 ml) and heated to reflux for 48 hours. The reaction mixture was lyophilized and then chromatographed on reverse phase HPLC with a column of Whatman Partisil $40^R$ (ODS-3) (25 x 500 mm) at a flow rate of 15 ml/minute. The column was eluted with 15 percent acetonitrile - 0.1 M pH 3.2 phosphate buffer (2000 ml) and 18 percent acetonitrile - buffer solution (1000 ml). The appropriate fractions were combined, lyophilized and desalted as described below to yield the desired AAD 216 pseudoaglycone.

The desalting procedure involved a pooling of the appropriate fractions from the HPLC and removal of the acetonitrile at reduced pressure. The resulting aqueous samples were loaded onto an XAD-7 resin column and eluted with deionized water until the conductivity of the outflow was less than 1.5 MHO. The column was then eluted with aqueous acetonitrile (50%) and the eluant lyophilized to afford the desired products.

EXAMPLE 12

Preparation of AAD 216 aglycone and AAD 216A pseudoaglycone

AAD 216A (0.74 g.) was dissolved in 5 percent concentrated hydrochloric acid and dimethylsulfoxide (40 ml) and heated for 15 minutes at 100° C. The reaction mixture was diluted with 14 percent acetonitrile 0.1 M pH 3.2 phosphate buffer and chromatographed with the equipment described in Example 1. The column was eluted with 14 percent acetonitrile-buffer solution (1000 ml) followed by 18 percent (500 ml), 22 percent (500 ml), 26 percent (500 ml) and 30 percent (1000 ml) acetonitrile-buffer solution. The appropriate fractions containing AAD 216A pseudoaglycone were combined and the appropriate fractions containing AAD 216 aglycone were combined. Both of the combined fractions were separately lyophilized and desalted using the procedure in Example 2 to afford the desired products.

EXAMPLE 13

Preparation of AAD 216B Pseudoaglycone

AAD 216B (1.64 g) was dissolved in dimethylsulfoxide (40 ml) and concentrated hydrochloric acid (2 ml) was added. The reaction mixture was heated at 100° C for 15 minutes and then diluted with 31 percent acetonitrile 0.1 M pH 6.0 phosphate buffer (450 ml) which was loaded onto an affinity chromatography column (700 ml - Affigel 10 D-ala-D-ala). The column was washed with 0.1 M pH 6.0 phosphate buffer (1000 ml), water (2000 ml) and 1 percent aqueous acetonitrile (1000 ml). The reaction products were eluted off the affinity chromatography column with 50 percent acetonitrile in 0.15 N ammonia (2000 ml). The eluant was lyophilized, dissolved in 10 percent acetonitrile in 0.1 M pH 6.0 phosphate buffer and chromatographed on the equipment described in Example 1. The column was eluted with 10 percent acetonitrile in 0.1 M pH 6.0 phosphate buffer (500 ml), followed by 15 percent (750 ml), 18 percent (750 ml), 22 percent (500 ml), 26 percent (50 ml), 30 percent (500 ml) and 35 percent (1000 ml) acetonitrile buffer solution. The appropriate factions containing AAD 216B pseudo-aglycone were combined and the fractions containing AAD 216 aglycone were combined. Both combined fractions were separately lyophilized and desalted using the procedure in Example 7 to afford the desired products.

Utilizing substantially the procedures of Examples 11, 12 and 13, the remaining naturally occurring AAD 216 factors are hydrolyzed to yield the factor pseudoaglycones, the mannosyl pseudoaglycone and the aglycone.

**Claims**

**Claims for the following Contracting States : BE, CH, DE, FR, GB, IT, LI, LU, NL, SE**

**1.** An AAD 216 antibiotic which has the following structural formula

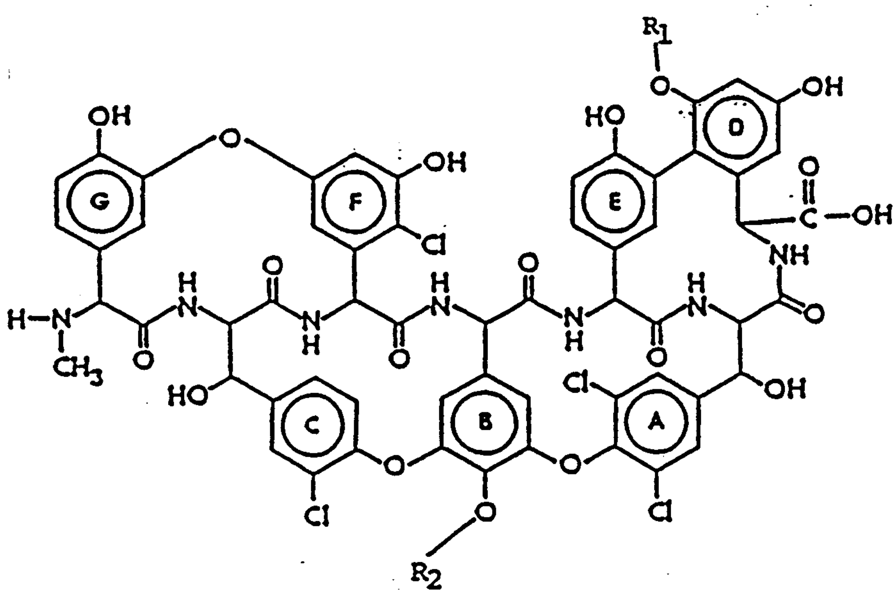

(I)

or a desoxy analog thereof, wherein $R_1$ is mannosyl or hydrogen and $R_2$ is hydrogen or

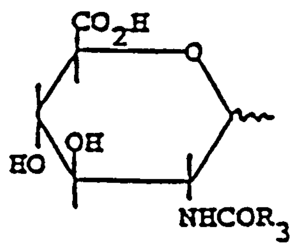

(II)

wherein $R_3$ is a $C_{8-12}$ alkyl or alkenyl, branched or linear, optionally substituted by a hydroxyl, provided that when the antibiotic is not the desoxy analog, $R_2$ is not hydrogen and $R_3$ is not $-(CH_2)_8-CH_3$, $-(CH_2)_7-CH(CH_3)_2$ or $-(CH_2)_8-CH(CH_3)_2$, and with the proviso that the antibiotic is not in combination with AAD 216A, AAD 216B and AAD 216C.

**2.** An antibiotic of claim 1 which is an individual pure antibiotic.

**3.** An antibiotic of claim 1 or 2 selected from the group consisting of AAD 216 factors F, G, H, J, K, L, M, N, O, P, Q, R, S, D, B1, B2, B3, B4, B5, C1, C2, C3, C4, C5 and C6 wherein each factor has the structure represented by formula (I), except that factors B1, B5, C3, C4, and factors comprising factor M are desoxy analogs of the core structure, and wherein the factors are distinguishable by retention time in HPLC (HPLC-RT), using a Beckman Ultrasphere ODS column and using a gradient of 25 to 40% acetonitrile in 0.1 M phosphoric acid (pH 3.2), or by retention time of methyl esters of $R_3COOH$ in gas chromatography (GC-RT), using a Quadrex glass capillary, Type 007-OV17 column in an oven programmed from 60° C to 240° C at a rate of 10° C per minute, as shown in the following table,

| Factor | HPLC RT (minutes) | GC-RT (minutes) | PREDICTED FORMULA ($R_3CO_2H$) |
|---|---|---|---|
| F1 | 5.9 | 11.8 | $C_{11}$,OH |
| 2 | | 11.9 | $C_{11}$,OH,olefin |
| 3 | | 12.1 | $C_{11}$,OH |
| G1 | 7.7 | 10.8 | $C_{12}$, olefin |
| 2 | | 12.9 | $C_{12}$,OH |
| 3 | | 13.0 | $C_{12}$,OH |
| H | 8.4 | 13.8 | ) |
| J | 8.7 | 13.3 | ) |
| K | 9.1 | 13.8 | ) $C_{12}$,OH |
| L1 | 9.2 | 13.3 | ) |
| 2 | | 13.6 | ) |
| 3 | | 13.8 | $C_{12}$,OH,olefin |
| M1 | 10.1 | 14.1 | $C_{13}$,OH |
| 2 | | 14.1 | $C_{13}$,OH |
| 3 | | 13.3 | $C_{12}$,OH |
| N | 10.6 | 14.1 | ) |
| O | 10.7 | 14.6 | ) $C_{13}$,OH |
| P1 | 11.4 | 14.5 | ) |
| 2 | | 9.7 | $C_{10}$,OH |
| 3 | | 6.5 | $C_9$ |

| Factor | HPLC RT (minutes) | GC-RT (minutes) | PREDICTED FORMULA ($R_3CO_2H$) |
|---|---|---|---|
| Q | 11.7 | 9.7 | $C_{10}$,OH |
| D | 12.8 | 7.9 | $C_{10}$,olefin |
| R | 13.6 | 8.2 | $C_{10}$,branched,olefin |
| S | 14.0 | 7.4 | $C_{10}$,branched |
| B1 | 16.3 | 7.9 | $C_{10}$ |
| B2 | 16.7 | 8.9 | $C_{11}$,branched |
| B3-4 | 17.8 | 9.3 | $C_{11}$ |
| | 17.9 | 10.0 | $C_{11}$,branched |
| B5 | 18.8 | 8.7 | $C_{11}$,branched |
| C1 | 19.6 | 10.2 | $C_{12}$,branched |
| C2 | 20.1 | 10.6 | $C_{12}$ |
| C3-4 | 20.7 | 10.0 | $C_{12}$,branched |
| | 20.8 | 10.6 | $C_{12}$ |
| C5-6 | 21.0 | 11.4 | $C_{13}$,branched |
| | 21.3 | 11.3 | $C_{13}$,branched |

**4.** Antibiotic of any one of claims 1 to 3 wherein a desoxy analogue is an anlogue which lacks a benzylic hydroxyl group.

**5.** An antibiotic mixture comprising as its only active components antibiotics of claim 3.

**6.** An antibiotic mixture of claim 5 comprising AAD 216 factors D, F, G, H, J, K, L, M, N, O, P and Q as defined in claim 3.

**7.** A process for the manufacture of an antibiotic compound of any one of claims 1 to 4, which comprises culturing Kibdelosporangium aridum Shearer gen. nov., sp. nov. ATCC 39323 microorganisms or mutants or variants thereof which retain the ability to produce the claimed antibiotics in an aqueous nutrient medium containing assimilable sources of nitrogen and carbon under submerged aerobic conditions, isolating the antibiotic compound in a purified or partially purified state, and optionally hydrolysing the antibiotic compound.

**8.** A process for the preparation of an antibiotic compound of any one of claims 1 to 4 wherein $R_1$ is hydrogen which comprises the partial acidic hydrolysis of an AAD 216 antibiotic compound therein $R_1$ is mannosyl.

**9.** A composition comprising one or more antibiotic compounds of any one of claims 1 to 4 and a pharmaceutically acceptable carrier.

**10.** An animal feed composition comprising a nontoxic amount of one or more antibiotic compounds of any one of claims 1 to 4 which is effective in increasing the feed efficiency of meat or milk producing animals in an animal feed.

**11.** An animal feed premix composition comprising a premix vehicle and from 5-75% by weight of one or more antibiotic compounds of any one of claims 1 to 4.

**12.** A method for improving the efficiency of feed utilization in an animal comprising orally administering to the animal an effective amount of one or more antibiotic compounds of any one of claims 1 to 4 or of the composition of claim 10.

**13.** A method for improving milk production in a lactating ruminant animal comprising orally administering to the animal an effective amount of one or more antibiotic compounds of any one of claims 1 to 4 or of the composition of claim 10.

**Claims for the following Contracting State : AT**

**1.** A process for the manufacture of an AAD 216 antibiotic which has the following structural formula

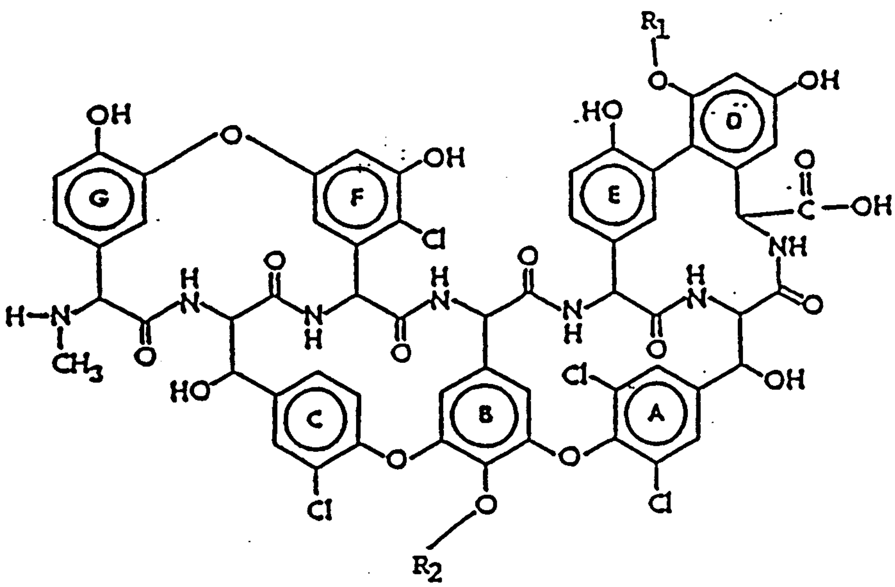

(I)

or a desoxy analog thereof, wherein $R_1$ is mannosyl or hydrogen and $R_2$ is hydrogen or

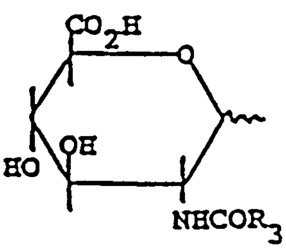

(II)

wherein $R_3$ is a $C_{8-12}$ alkyl or alkenyl, branched or linear, optionally substituted by a hydroxyl, provided that when the antibiotic is not the desoxy analog, $R_2$ is not hydrogen and $R_3$ is not $-(CH_2)_8-CH_3$, $-(CH_2)_7-CH(CH_3)_2$ or $-(CH_2)_8-CH(CH_3)_2$ and with the proviso that the antibiotic is not in combination with AAD 216A, AAD 216B and AAD 216C, which process comprises culturing Kibdelosporangium aridum Shearer gen. nov., sp. nov. ATCC 39323 microorganisms or mutants or variants thereof in an aqueous nutrient medium containing assimilable sources of nitrogen and carbon under submerged aerobic conditions, isolating the antibiotic compound, in a purified or partially purified state, and optionally hydrolysing the antibiotic compound.

**2.** A process according to claim 1 for preparing an antibiotic of claim 1 which is a pure individual antibiotic.

**3.** A process according to claim 1 or 2 for preparing an antibiotic selected from the group consisting of

AAD 216 factors F, G, H, J, K, L, M, N, O, P, Q, R, S, D, B1, B2, B3, B4, B5, C1, C2, C3, C4, C5 and C6 wherein each factor has the structure represented by formula (I), except that factors B1, B5, C3, C4, and factors comprising factor M are desoxy analogs of the core structure, and wherein the factors are distinguishable by retention time in HPLC (HPLC-RT), using a Beckman Ultrasphere ODS column and using a gradient of 25 to 40% acetonitrile in 0.1 M phosphoric acid (pH 3.2), or by retention time of methyl esters of $R_3COOH$ in gas chromatography (GC-RT), using a Quadrex glass capillary, Type 007-OV17 column in an oven programmed from 60° C to 240° C at a rate of 10° C per minute, as shown in the following table,

| Factor | HPLC RT (minutes) | GC-RT (minutes) | PREDICTED FORMULA ($R_3CO_2H$) |
|---|---|---|---|
| F1 | 5.9 | 11.8 | $C_{11}$,OH |
| 2 | | 11.9 | $C_{11}$,OH,olefin |
| 3 | | 12.1 | $C_{11}$,OH |
| G1 | 7.7 | 10.8 | $C_{12}$,olefin |
| 2 | | 12.9 | $C_{12}$,OH |
| 3 | | 13.0 | $C_{12}$,OH |
| H | 8.4 | 13.8 | ) |
| J | 8.7 | 13.3 | ) $C_{12}$,OH |
| K | 9.1 | 13.8 | ) |

| Factor | HPLC RT (minutes) | GC-RT (minutes) | PREDICTED FORMULA ($R_3CO_2H$) |
|---|---|---|---|
| L1 | 9.2 | 13.3 | ) |
| 2 | | 13.6 | ) $C_{12}$,OH |
| 3 | | 13.8 | $C_{12}$,OH,olefin |
| M1 | 10.1 | 14.1 | $C_{13}$,OH |
| 2 | | 14.1 | $C_{13}$,OH |
| 3 | | 13.3 | $C_{12}$,OH |
| N | 10.6 | 14.1 | ) |
| O | 10.7 | 14.6 | ) $C_{13}$,OH |
| P1 | 11.4 | 14.5 | ) |
| 2 | | 9.7 | $C_{10}$,OH |
| 3 | | 6.5 | $C_9$ |
| Q | 11.7 | 9.7 | $C_{10}$,OH |
| D | 12.8 | 7.9 | $C_{10}$,olefin |
| R | 13.6 | 8.2 | $C_{10}$,branched,olefin |
| S | 14.0 | 7.4 | $C_{10}$,branched |
| B1 | 16.3 | 7.9 | $C_{10}$ |
| B2 | 16.7 | 8.9 | $C_{11}$,branched |
| B3-4 | 17.8 | 9.3 | $C_{11}$ |
| | 17.9 | 10.0 | $C_{11}$,branched |
| B5 | 18.8 | 8.7 | $C_{11}$,branched |
| C1 | 19.6 | 10.2 | $C_{12}$,branched |
| C2 | 20.1 | 10.6 | $C_{12}$ |
| C3-4 | 20.7 | 10.0 | $C_{12}$,branched |
| | 20.8 | 10.6 | $C_{12}$ |
| C5-6 | 21.0 | 11.4 | $C_{13}$,branched |
| | 21.3 | 11.3 | $C_{13}$,branched |

**4.** A process according to any one of claims 1 to 3 for preparing an antibiotic wherein a desoxy analogue is an analogue which lacks a benzylic hydroxyl group.

**5.** A process for the preparation of an antibiotic compound of any one of claims 1 to 4 wherein $R_1$ is hydrogen which comprises the partial acidic hydrolysis of an AAD 216 antibiotic compound wherein $R_1$ is mannosyl.

**6.** A process for preparing a composition comprising the admixing of one or more antibiotic compounds as defined in any one of claims 1 to 4 and a pharmaceutically acceptable carrier.

**7.** A process for preparing an animal feed composition comprising the admixing of one or more antibiotic

compounds as defined in any one of claims 1 to 4 with an animal feed.

8. A process for preparing an animal feed premix composition comprising the admixing of one or more antibiotic compounds as defined in any one of claims 1 to 4 with a premix vehicle.

9. A method for improving the efficiency of feed utilization in an animal comprising orally administering to the animal an effective amount of one or more antibiotic compounds as defined in any one of claims 1 to 4 or of the composition as defined in claim 7.

10. A method for improving milk production in a lactating ruminant animal comprising orally administering to the animal an effective amount of one or more antibiotic compounds as defined in any one of claims 1 to 4 or of the composition as defined in claim 7.

**Revendications**

**Revendications pour les Etats contractants suivants : BE, CH, DE, FR, GB, IT, LI, LU, NL, SE**

1. Antibiotique AAD 216 qui a la formule développée suivante :

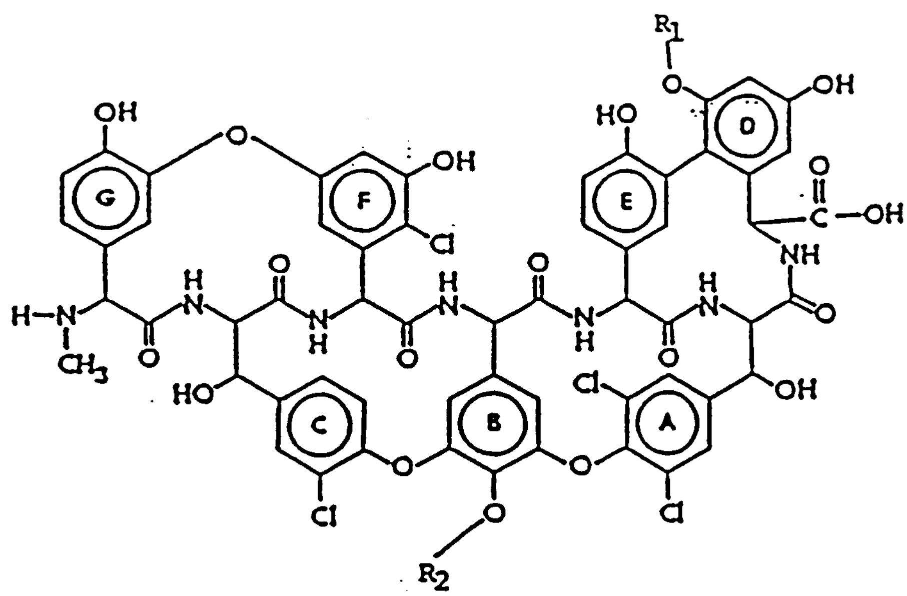

(I)

ou un analogue désoxy de celui-ci, où $R_1$ est un groupe mannosyle ou un atome d'hydrogène et $R_2$ est un atome d'hydrogène ou

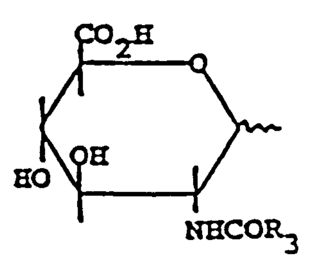

(II)

où $R_3$ est un groupe alkyle ou alcényle en $C_{8-12}$ ramifié ou linéaire, éventuellement substitué par un groupe hydroxyle, sous réserve que lorsque l'antibiotique n'est pas l'analogue désoxy, $R_2$ n'est pas un atome d'hydrogène et $R_3$ n'est pas $-(CH_2)_8-CH_3$, $-(CH_2)_7-CH(CH_3)_2$ ou $-(CH_2)_8-CH(CH_3)_2$, et sous réserve que l'antibiotique ne soit pas en association avec l'AAD 216A, l'AAD 216B et l'AAD 216C.

2. Antibiotique selon la revendication 1 qui est un antibiotique individuel pur.

3. Antibiotique selon la revendication 1 ou 2 choisi parmi le groupe des facteurs AAD 216 F, G, H, J, K, L,

M, N, O, P, Q, R, S, D, B1, B2, B3, B4, B5, C1, C2, C3, C4, C5 et C6, où chaque facteur a la structure représentée par la formule (I), si ce n'est que les facteurs B1, B5, C3, C4 et les facteurs comprenant le facteur M sont des analogues désoxy de la structure centrale, et où les facteurs peuvent être différenciés par le temps de rétention en CLHP (CLHP-TR) par emploi d'une colonne d'Ultrasphere ODS de Beckman et d'un gradient de 25 à 40 % d'acétonitrile dans de l'acide phosphorique 0,1 M (pH 3,2) ou par le temps de rétention des esters méthyliques de $R_3COOH$ en chromatographie gazeuse (CG-TR) par emploi d'une colonne capillaire de verre Quadrex Type 007-OV17, dans un four programmé de 60° C à 240° C à la vitesse de 10° C/min, comme indiqué dans le tableau suivant :

| Facteur | CLHP-TR (min) | CG-TR (min) | Formule prévue ($R_3CO_2H$) |
|---|---|---|---|
| F1 | 5,9 | 11,8 | $C_{11}$, OH |
| 2 | | 11,9 | $C_{11}$, OH, oléfine |
| 3 | | 12,1 | $C_{11}$, OH |
| G1 | 7,7 | 10,8 | $C_{12}$, oléfine |
| 2 | | 12,9 | $C_{12}$, OH |
| 3 | | 13,0 | $C_{12}$, OH |
| H | 8,4 | 13,8 | ) |
| J | 8,7 | 13,3 | ) |
| K | 9,1 | 13,8 | ) $C_{12}$, OH |
| L1 | 9,2 | 13,3 | ) |
| 2 | | 13,6 | ) |
| 3 | | 13,8 | $C_{12}$, OH, oléfine |
| M1 | 10,1 | 14,1 | $C_{13}$, OH |
| 2 | | 14,1 | $C_{13}$, OH |
| 3 | | 13,3 | $C_{12}$, OH |
| N | 10,6 | 14,1 | ) |
| O | 10,7 | 14,6 | ) $C_{13}$, OH |
| P1 | 11,4 | 14,5 | ) |
| 2 | | 9,7 | $C_{10}$, OH |
| 3 | | 6,5 | $C_9$ |

| | | | |
|---|---|---|---|
| Q | 11,7 | 9,7 | $C_{10}$, OH |
| D | 12,8 | 7,9 | $C_{10}$, oléfine |
| R | 13,6 | 8,2 | $C_{10}$, ramifié, oléfine |
| S | 14,0 | 7,4 | $C_{10}$, ramifié |
| B1 | 16,3 | 7,9 | $C_{10}$ |
| B2 | 16,7 | 8,9 | $C_{11}$, ramifié |
| B3-4 | 17,8 | 9,3 | $C_{11}$ |
| | 17,9 | 10,0 | $C_{11}$, ramifié |
| B5 | 18,8 | 8,7 | $C_{11}$, ramifié |
| C1 | 19,6 | 10,2 | $C_{12}$, ramifié |
| C2 | 20,1 | 10,6 | $C_{12}$ |
| C3-4 | 20,7 | 10,0 | $C_{12}$, ramifié |
| | 20,8 | 10,6 | $C_{12}$ |
| C5-6 | 21,0 | 11,4 | $C_{13}$, ramifié |
| | 21,3 | 11,3 | $C_{13}$, ramifié |

**4.** Antibiotique selon l'une quelconque des revendications 1 à 3, dans lequel un analogue désoxy est un analogue auquel manque un groupe hydroxyle benzylique.

**5.** Mélange d'antibiotiques comprenant comme seuls composants actifs les antibiotiques de la revendication 3.

**6.** Mélange d'antibiotiques selon la revendication 5 comprenant les facteurs AAD 216 D, F, G, H, J, K, L, M, N, O, P et Q, comme définis dans la revendication 3.

**7.** Procédé pour la préparation d'un composé antibiotique selon l'une quelconque des revendications 1 à 4, qui comprend la culture de microorganismes Kibdelosporangium aridum Shearer gen. nov., sp. nov. ATCC 39323 ou de mutants ou de variants de ceux-ci conservant la capacité de produire les antibiotiques revendiqués dans un milieu nutritif aqueux contenant des sources assimilables d'azote et de carbone dans des conditions aérobies submergées, l'isolement du composé antibiotique sous une forme purifiée ou partiellement purifiée et, éventuellement l'hydrolyse du composé antibiotique.

**8.** Procédé pour la préparation d'un composé antibiotique selon l'une quelconque des revendications 1 à 4 dans lequel $R_1$ est un atome d'hydrogène, qui comprend l'hydrolyse acide partielle d'un composé antibiotique AAD 216 dans lequel $R_1$ est un groupe mannosyle.

**9.** Composition comprenant un ou plusieurs composés antibiotiques selon l'une quelconque des revendications 1 à 4 et un véhicule pharmaceutiquement acceptable.

**10.** Composition alimentaire pour animaux comprenant une quantité non toxique d'un ou plusieurs composés antibiotiques selon l'une quelconque des revendications 1 à 4, qui accroît l'efficacité alimentaire des animaux producteurs de viande ou de lait dans un aliment pour animal.

**11.** Composition de prémélange alimentaire pour animaux comprenant un véhicule de prémélange et 5 à 75 % en poids d'un ou plusieurs composés antibiotiques selon l'une quelconque des revendications 1 à 4.

12. Procédé pour améliorer l'efficacité de l'utilisation des aliments d'un animal comprenant l'administration orale à l'animal d'une quantité efficace d'un ou plusieurs composés antibiotiques selon l'une quelconque des revendications 1 à 4 ou de la composition de la revendication 10.

13. Procédé pour améliorer la production laitière d'un ruminant en état de lactation comprenant l'administration orale à l'animal d'une quantité efficace d'un ou plusieurs composés antibiotiques selon l'une quelconque des revendications 1 à 4 ou de la composition de la revendication 10.

**Revendications pour l'Etat contractant suivant : AT**

1. Procédé pour la préparation d'un antibiotique AAD 216 qui a la formule développée suivante :

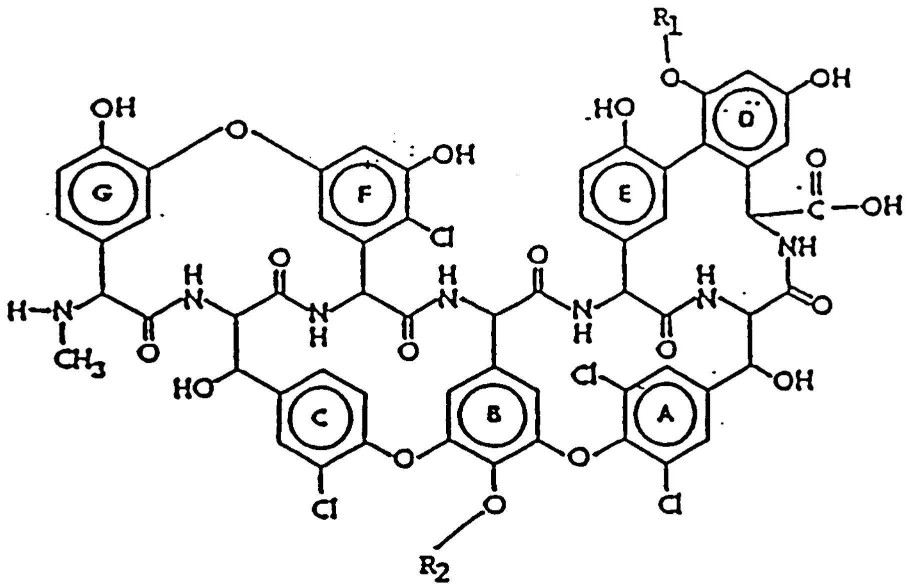

(I)

ou un analogue désoxy de celui-ci, où $R_1$ est un groupe mannosyle ou un atome d'hydrogène et $R_2$ est un atome d'hydrogène ou

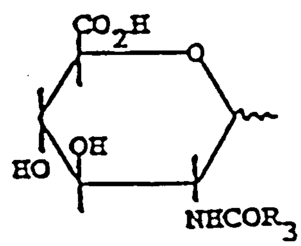

(II)

où $R_3$ est un groupe alkyle ou alcényle en $C_{8-12}$ ramifié ou linéaire, éventuellement substitué par un groupe hydroxyle, sous réserve que lorsque l'antibiotique n'est pas l'analogue désoxy, $R_2$ n'est pas un atome d'hydrogène et $R_3$ n'est pas $-(CH_2)_8-CH_3$, $-(CH_2)_7-CH(CH_3)_2$ ou $-(CH_2)_8-CH(CH_3)_2$, et sous réserve que l'antibiotique ne soit pas en association avec l'AAD 216A, l'AAD 216B et l'AAD 216C, lequel procédé comprend la culture de microorganismes Kibdelosporangium aridum Shearer gen. nov., sp. nov. ATCC 39323 ou de mutants ou de variants de ceux-ci dans un milieu nutritif aqueux contenant des sources assimilables d'azote et de carbone dans des conditions aérobies submergées, l'isolement du composé antibiotique sous une forme purifiée ou partiellement purifiée et éventuellement l'hydrolyse du composé antibiotique.

2. Procédé selon la revendication 1 pour préparer un antibiotique selon la revendication 1 qui est un antibiotique individuel pur.

3. Procédé selon la revendication 1 ou 2 pour préparer un antibiotique choisi parmi le groupe des facteurs AAD 216 F, G, H, J, K, L, M, N, O, P, Q, R, S, D, B1, B2, B3, B4, B5, C1, C2, C3, C4, C5 et C6, où chaque facteur a la structure représentée par la formule (I), si ce n'est que les facteurs B1, B5, C3, C4 et les facteurs comprenant le facteur M sont des analogues désoxy de la structure centrale, et où les facteurs peuvent être différenciés par le temps de rétention en CLHP (CLHP-TR) par emploi d'une colonne d'Ultrasphere ODS de Beckman et d'un gradient de 25 à 40 % d'acétonitrile dans de l'acide phosphorique 0,1 M (pH 3,2) ou par le temps de rétention des esters méthyliques de $R_3COOH$ en chromatographie gazeuse (CG-TR) par emploi d'une colonne capillaire de verre Quadrex Type 007-OV17, dans un four programmé de 60 °C à 240 °C à la vitesse de 10 °C/min, comme indiqué dans le tableau suivant :

| Facteur | CLHP-TR (min) | CG-TR (min) | Formule prévue ($R_3CO_2H$) |
|---|---|---|---|
| F1 | 5,9 | 11,8 | $C_{11}$, OH |
| 2 | | 11,9 | $C_{11}$, OH, oléfine |
| 3 | | 12,1 | $C_{11}$, OH |
| G1 | 7,7 | 10,8 | $C_{12}$, oléfine |
| 2 | | 12,9 | $C_{12}$, OH |
| 3 | | 13,0 | $C_{12}$, OH |
| H | 8,4 | 13,8 | ) |
| J | 8,7 | 13,3 | ) |
| K | 9,1 | 13,8 | ) $C_{12}$, OH |
| L1 | | 13,3 | ) |
| 2 | | 13,6 | ) |
| 3 | | 13,8 | ) |

| | | | |
|---|---|---|---|
| M1 | 10,1 | 14,1 | $C_{13}$, OH |
| 2 | | 14,1 | $C_{13}$, OH |
| 3 | | 13,3 | $C_{12}$, OH |
| N | 10,6 | 14,1 | ) |
| O | 10,7 | 14,6 | ) $C_{13}$, OH |
| P1 | 11,4 | 14,5 | ) |
| 2 | | 9,7 | $C_{10}$, OH |
| 3 | | 6,5 | $C_9$ |
| Q | 11,7 | 9,7 | $C_{10}$, OH |
| D | 12,8 | 7,9 | $C_{10}$, oléfine |
| R | 13,6 | 8,2 | $C_{10}$, ramifié, oléfine |
| S | 14,0 | 7,4 | $C_{10}$, ramifié |
| B1 | 16,3 | 7,9 | $C_{10}$ |
| B2 | 16,7 | 8,9 | $C_{11}$, ramifié |
| B3-4 | 17,8 | 9,3 | $C_{11}$ |
| | 17,9 | 10,0 | $C_{11}$, ramifié |
| B5 | 18,8 | 8,7 | $C_{11}$, ramifié |
| C1 | 19,6 | 10,2 | $C_{12}$, ramifié |
| C2 | 20,1 | 10,6 | $C_{12}$ |
| C3-4 | 20,7 | 10,0 | $C_{12}$, ramifié |
| | 20,8 | 10,6 | $C_{12}$ |
| C5-6 | 21,0 | 11,4 | $C_{13}$, ramifié |
| | 21,3 | 11,3 | $C_{13}$, ramifié |

**4.** Procédé selon l'une quelconque des revendications 1 à 3 pour préparer un antibiotique dans lequel un analogue désoxy est un analogue auquel manque un groupe hydroxyle benzylique.

**5.** Procédé pour la préparation d'un composé antibiotique selon l'une quelconque des revendications 1 à 4 dans lequel $R_1$ est un atome d'hydrogène, qui comprend l'hydrolyse acide partielle d'un composé antibiotique AAD 216 dans lequel $R_1$ est un groupe mannosyle.

**6.** Procédé pour préparer une composition comprenant le mélange d'un ou plusieurs composés antibiotiques selon l'une quelconque des revendications 1 à 4 et d'un véhicule pharmaceutiquement acceptable.

**7.** Procédé pour préparer une composition alimentaire pour animaux, comprenant le mélange d'un ou plusieurs composés antibiotiques comme définis dans l'une quelconque des revendications 1 à 4 avec un aliment pour animaux.

**8.** Procédé pour préparer une composition de prémélange alimentaire pour animaux, comprenant le mélange d'un ou plusieurs composés antibiotiques comme définis dans l'une quelconque des revendications 1 à 4 avec un véhicule de prémélange.

**9.** Procédé pour améliorer l'efficacité de l'utilisation des aliments d'un animal, comprenant l'administration orale à l'animal d'une quantité efficace d'un ou plusieurs composés antibiotiques selon l'une quelconque des revendications 1 à 4 ou de la composition de la revendication 7.

**10.** Procédé pour améliorer la production laitière d'un ruminant en état de lactation, comprenant l'administration orale à l'animal d'une quantité efficace d'un ou plusieurs composés antibiotiques selon l'une quelconque des revendications 1 à 4 ou de la composition de la revendication 7.

**Patentansprüche**

**Patentansprüche für folgende Vertragsstaaten : BE, CH, DE, FR, GB, IT, LI, LU, NL, SE**

**1.** AAD 216-Antibiotikum mit der folgenden Strukturformel

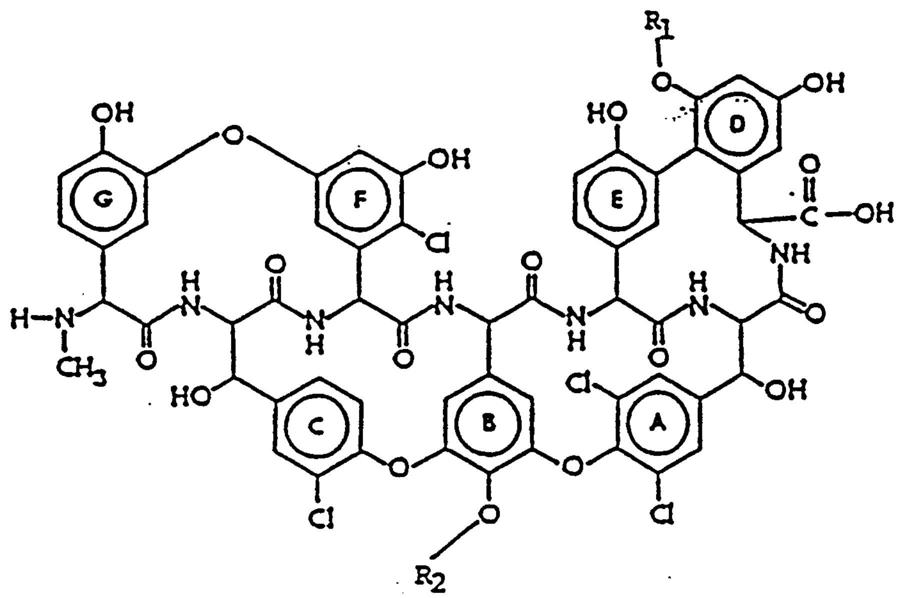

(I)

oder ein Desoxyanalog davon, wobei $R_1$ eine Mannosylgruppe oder ein Wasserstoffatom ist und $R_2$ ein Wasserstoffatom oder ein Rest der Formel

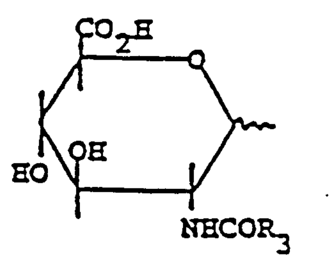

(II)

ist, in der $R_3$ ein verzweigtkettiger oder geradkettiger $C_{8-12}$-Alkyl- oder -Alkenylrest ist, gegebenenfalls substituiert mit einer Hydroxylgruppe, mit der Maßgabe, daß, wenn das Antibiotikum nicht das Desoxyanalog ist, $R_2$ kein Wasserstoffatom ist und $R_3$ kein Rest der Formel $-(CH_2)_8-CH_3$, $-(CH_2)_7-CH-(CH_3)_2$ oder $-(CH_2)_8-CH(CH_3)_2$ ist, und mit der Maßgabe, daß das Antibiotikum nicht mit AAD 216A, AAD 216B und AAD 216C kombiniert wird.

**2.** Antibiotikum nach Anspruch 1, das ein einzelnes reines Antibiotikum ist.

**3.** Antibiotikum nach Anspruch 1 oder 2, ausgewählt aus der Gruppe von AAD 216-Faktoren F, G, H, J, K, L, M, N, O, P, Q, R, S, D, B1, B2, B3, B4, B5, C1, C2, C3, C4, C5 und C6, wobei jeder Faktor die durch Formel (I) dargestellte Struktur aufweist, mit der Ausnahme, daß die Faktoren B1, B5, C3, C4 und

Faktor M umfassende Faktoren Desoxyanaloga der Kernstruktur sind, und wobei die Faktoren durch die Retentionszeit in der HPLC (HPLC-RT) mit einer Beckman Ultrasphere ODS-Säule und mit einem Gradienten von 25 bis 40 % Acetonitril in 0,1 M Phosphorsäure (pH 3,2) oder durch die Retentionszeit der Methylester von $R_3COOH$ in der Gaschromatographie (GC-RT) mit einer Quadrex-Glaskapillarsäule, Typ 007-OV17, in einem von 60°C bis 240°C bei einer Geschwindigkeit von 10°C pro Minute programmierten Ofen, wie in der folgenden Tabelle gezeigt, unterscheidbar sind:

| Factor | HPLC RT (Minuten) | GC-RT (Minuten) | vorausgesagte Formel ($R_3CO_2H$) |
|---|---|---|---|
| F1 | 5.9 | 11.8 | $C_{11}$,OH |
| 2 | | 11.9 | $C_{11}$,OH, Olefin |
| 3 | | 12.1 | $C_{11}$,OH |
| G1 | 7.7 | 10.8 | $C_{12}$, Olefin |
| 2 | | 12.9 | $C_{12}$,OH |
| 3 | | 13.0 | $C_{12}$,OH |
| H | 8.4 | 13.8 | ) |
| J | 8.7 | 13.3 | ) |
| K | 9.1 | 13.8 | ) $C_{12}$,OH |
| L1 | 9.2 | 13.3 | ) |
| 2 | | 13.6 | ) |
| 3 | | 13.8 | $C_{12}$,OH, Olefin |
| M1 | 10.1 | 14.1 | $C_{13}$,OH |

| Factor | HPLC RT (Minuten) | GC-RT (Minuten) | vorausgesagte Formel ($R_3CO_2H$) |
|---|---|---|---|
| 2 | | 14.1 | $C_{13}$, OH |
| 3 | | 13.3 | $C_{12}$, OH |
| N | 10.6 | 14.1 | ) |
| O | 10.7 | 14.6 | ) $C_{13}$, OH |
| P1 | 11.4 | 14.5 | ) |
| 2 | | 9.7 | $C_{10}$, OH |
| 3 | | 6.5 | $C_9$ |
| Q | 11.7 | 9.7 | $C_{10}$, OH |
| D | 12.8 | 7.9 | $C_{10}$, - Olefin |
| R | 13.6 | 8.2 | $C_{10}$, verzweigt, Olefin |
| S | 14.0 | 7.4 | $C_{10}$, verzweigt |
| B1 | 16.3 | 7.9 | $C_{10}$ |
| B2 | 16.7 | 8.9 | $C_{11}$, verzweigt |
| B3-4 | 17.8 | 9.3 | $C_{11}$ |
| | 17.9 | 10.0 | $C_{11}$, verzweigt |
| B5 | 18.8 | 8.7 | $C_{11}$, verzweigt |
| C1 | 19.6 | 10.2 | $C_{12}$, verzweigt |
| C2 | 20.1 | 10.6 | $C_{12}$ |
| C3-4 | 20.7 | 10.0 | $C_{12}$, verzweigt |
| | 20.8 | 10.6 | $C_{12}$ |
| C5-6 | 21.0 | 11.4 | $C_{13}$, verzweigt |
| | 21.3 | 11.3 | $C_{13}$, verzweigt |

**4.** Antibiotikum nach einem der Ansprüche 1 bis 3, wobei dem Desoxyanalog eine Benzylhydroxylgruppe fehlt.

**5.** Antibiotisches Gemisch, das als einzige wirksame Komponenten Antibiotika von Anspruch 3 umfaßt.

**6.** Antibiotisches Gemisch nach Anspruch 5, das die AAD 216-Faktoren D, F, G, H, J, K, L, M, N, O, P und Q, wie in Anspruch 3 definiert, umfaßt.

**7.** Verfahren zur Herstellung einer antibiotischen Verbindung nach einem der Ansprüche 1 bis 4, umfassend die Züchtung von Kibdelosporangium aridum Shearer gen. nov., sp. nov. ATCC 39323 Mikroorganismen oder deren Mutanten oder Varianten, die die Fähigkeit zur Erzeugung der beanspruchten Antibiotika in einem wäßrigen, assimilierbare Stickstoff- und Kohlenstoffquellen enthaltendem Nährmedium unter aeroben Submersbedingungen aufweisen, die Isolierung der antibiotischen Verbindung in einem gereinigten oder teilweise gereinigtem Zustand und gegebenenfalls die Hydrolyse der antibiotischen Verbindung.

**8.** Verfahren zur Herstellung einer antibiotischen Verbindung nach einem der Ansprüche 1 bis 4, wobei $R_1$ ein Wasserstoffatom ist, das die teilweise saure Hydrolyse einer AAD 216 antibiotischen Verbindung, in der $R_1$ eine Mannosylgruppe ist, umfaßt.

**9.** Zusammensetzung, umfassend eine oder mehrere antibiotische Verbindungen nach einem der Ansprüche 1 bis 4 und einen pharmazeutisch verträglichen Träger.

**10.** Tierfutterzusammensetzung, umfassend eine nicht-toxische Menge einer oder mehrerer antibiotischer Verbindungen nach einem der Ansprüche 1 bis 4, die zur Steigerung des Futterwirkungsgrads von Fleisch- oder Milch- erzeugenden Tieren in einem Tierfutter wirksam ist.

**11.** Tierfutterzusammensetzungs-Vorgemisch, umfassend einen Vorgemisch-Träger und 5-75 Gew.-% eines oder mehrerer antibiotischer Verbindungen nach einem der Ansprüche 1 bis 4.

**12.** Verfahren zur Verbesserung des Wirkungsgrads der Futterverwertung in einem Tier, umfassend die orale Verabreichung einer wirksamen Menge eines oder mehrerer antibiotischer Verbindungen nach einem der Ansprüche 1 bis 4 oder der Zusammensetzung nach Anspruch 10 an das Tier.

**13.** Verfahren zur Verbesserung der Milcherzeugung bei einem milchgebenden Wiederkäuer, umfassend die orale Verabreichung einer wirksamen Menge einer oder mehrerer antibiotischer Verbindungen nach einem der Ansprüche 1 bis 4 oder der Zusammensetzung nach Anspruch 10 an das Tier.

**Patentansprüche für folgenden Vertragsstaat : AT**

**1.** Verfahren zur Herstellung eines AAD 216-Antibiotikums mit der folgenden Strukturformel

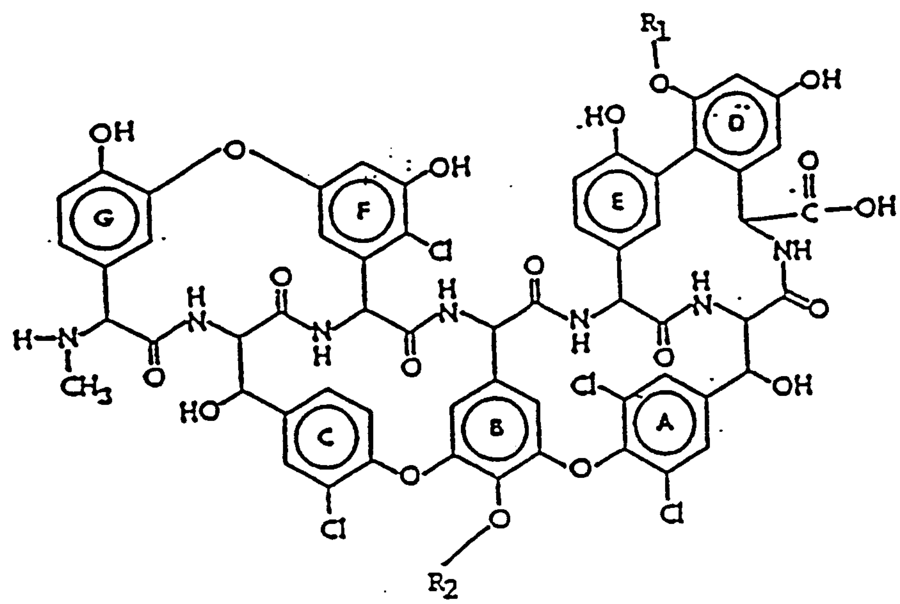

(I)

oder eines Desoxyanalogs davon, wobei $R_1$ eine Mannosylgruppe oder ein Wasserstoffatom ist und $R_2$ ein Wasserstoffatom oder ein Rest der Formel

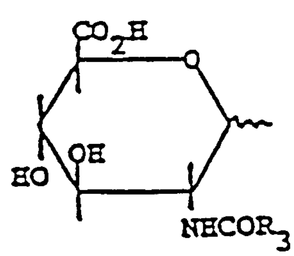

(II)

ist, in der $R_3$ ein verzweigtkettiger oder geradkettiger $C_{8-12}$-Alkyl- oder Alkenylrest ist, gegebenenfalls substituiert mit einer Hydroxylgruppe, mit der Maßgabe, daß, wenn das Antibiotikum nicht das

Desoxyanalog ist, $R_2$ kein Wasserstoffatom ist und $R_3$ kein Rest der Formel $-(CH_2)_8-CH_3$, $-(CH_2)_7-CH-(CH_3)_2$ oder $-(CH_2)_8-CH(CH_3)_2$ ist, und mit der Maßgabe, daß das Antibiotikum nicht mit AAD 216A, AAD 216B und AAD 216C kombiniert wird, wobei das Verfahren die Züchtung von Kibdelosporangium aridum Shearer gen. nov., sp. nov. ATCC 39323 Mikroorganismen oder deren Mutanten oder Varianten, die die Fähigkeit zur Erzeugung der beanspruchten Antibiotika in einem wäßrigen, assimilierbare Stickstoff- und Kohlenstoffquellen enthaltendem Nährmedium unter aeroben Submersbedingungen aufweisen, die Isolierung der antibiotischen Verbindung in einem gereinigten oder teilweise gereinigten Zustand und gegebenenfalls die Hydrolyse der antibiotischen Verbindung umfaßt.

**2.** Verfahren nach Anspruch 1 zur Herstellung eines Antibiotikums von Anspruch 1, das ein einziges reines Antibiotikum ist.

**3.** Verfahren nach Anspruch 1 oder 2 zur Herstellung eines Antibiotikums, ausgewählt aus der Gruppe von AAD 216-Faktoren F, G, H, J, K, L, M, N, O, P, Q, R, S, D, B1, B2, B3, B4, B5, C1, C2, C3, C4, C5 und C6, wobei jeder Faktor die durch Formel (I) dargestellte Struktur aufweist, mit der Ausnahme, daß die Faktoren B1, B5, C3, C4 und Faktor M umfassende Faktoren Desoxyanaloga der Kernstruktur sind und wobei die Faktoren durch die Retentionszeit in der HPLC (HPLC-RT) mit einer Beckman Ultrasphere ODS-Säule und mit einem Gradienten von 25 bis 40 % Acetonitril in 0,1 M Phosphorsäure (pH 3,2) oder durch die Retentionszeit der Methylester von $R_3COOH$ in der Gaschromatographie (GC-RT) mit einer Quadrex-Glaskapillarsäule, Typ 007-OV17, in einem von 60° C bis 240° C bei einer Geschwindigkeit von 10° C pro Minute programmierten Ofen, wie in der folgenden Tabelle gezeigt, unterscheidbar sind:

| Factor | HPLC RT (Minuten) | GC-RT (Minuten) | vorausgesagte Formel ($R_3CO_2H$) |
|---|---|---|---|
| F1 | 5.9 | 11.8 | $C_{11}$,OH |
| 2 | | 11.9 | $C_{11}$,OH, Olefin |
| 3 | | 12.1 | $C_{11}$,OH |
| G1 | 7.7 | 10.8 | $C_{12}$, Olefin |
| 2 | | 12.9 | $C_{12}$,OH |
| 3 | | 13.0 | $C_{12}$,OH |
| H | 8.4 | 13.8 | ) |
| J | 8.7 | 13.3 | ) $C_{12}$,OH |
| K | 9.1 | 13.8 | ) |
| L1 | 9.2 | 13.3 | ) |
| 2 | | 13.6 | ) $C_{12}$,OH |
| 3 | | 13.8 | $C_{12}$,OH, Olefin |
| M1 | 10.1 | 14.1 | $C_{13}$,OH |
| 2 | | 14.1 | $C_{13}$,OH |
| 3 | | 13.3 | $C_{12}$,OH |
| N | 10.6 | 14.1 | ) |
| O | 10.7 | 14.6 | ) $C_{13}$,OH |
| P1 | 11.4 | 14.5 | ) |
| 2 | | 9.7 | $C_{10}$,OH |
| 3 | | 6.5 | $C_9$ |
| Q | 11.7 | 9.7 | $C_{10}$,OH |
| D | 12.8 | 7.9 | $C_{10}$, Olefin |
| R | 13.6 | 8.2 | $C_{10}$, verzweigt,Olefin |
| S | 14.0 | 7.4 | $C_{10}$, verzweigt |
| B1 | 16.3 | 7.9 | $C_{10}$ |
| B2 | 16.7 | 8.9 | $C_{11}$, verzweigt |
| B3-4 | 17.8 | 9.3 | $C_{11}$ |
| | 17.9 | 10.0 | $C_{11}$, verzweigt |
| B5 | 18.8 | 8.7 | $C_{11}$, verzweigt |
| C1 | 19.6 | 10.2 | $C_{12}$, verzweigt |
| C2 | 20.1 | 10.6 | $C_{12}$ |
| C3-4 | 20.7 | 10.0 | $C_{12}$, verzweigt |
| | 20.8 | 10.6 | $C_{12}$ |
| C5-6 | 21.0 | 11.4 | $C_{13}$, verzweigt |
| | 21.3 | 11.3 | $C_{13}$, verzweigt |

**4.** Verfahren nach einem der Ansprüche 1 bis 3 zur Herstellung eines Antibiotikums, wobei dem Desoxyanalog eine Benzylhydroxylgruppe fehlt.

**5.** Verfahren zur Herstellung einer antibiotischen Verbindung nach einem der Ansprüche 1 bis 4, wobei $R_1$

ein Wasserstoffatom ist, das die teilweise saure Hydrolyse einer AAD 216 antibiotischen Verbindung, in der $R_1$ eine Mannosylgruppe ist, umfaßt.

**6.** Verfahren zur Herstellung einer Zusammensetzung, umfassend das Mischen einer oder mehrerer antibiotischer Verbindungen gemäß einem der Ansprüche 1 bis 4 und eines pharmazeutisch verträglichen Trägers.

**7.** Verfahren zur Herstellung einer Tierfutterzusammensetzung, umfassend das Mischen einer oder mehrerer antibiotischer Verbindungengemäß einem der Ansprüche 1 bis 4 mit einem Tierfutter.

**8.** Verfahren zur Herstellung eines Tierfutterzusammensetzungs-Vorgemisches, umfassend das Mischen einer oder mehrerer antibiotischer Verbindungen gemäß einem der Ansprüche 1 bis 4 mit einem Vorgemisch-Träger.

**9.** Verfahren zur Verbesserung des Wirkungsgrads der Futterverwertung in einem Tier, umfassend die orale Verabreichung einer wirksamen Menge einer oder mehrerer antibiotischer Verbindungen von einem der Ansprüche 1 bis 4 oder der Zusammensetzung von Anspruch 7 an das Tier.

**10.** Verfahren zur Verbesserung der Milcherzeugung bei einem milchgebenden Wiederkäuer, umfassend die orale Verabreichung einer wirksamen Menge eines oder mehrerer antibiotischer Verbindungen nach einem der Ansprüche 1 bis 4 oder der Zusammensetzung von Anspruch 7 an das Tier.

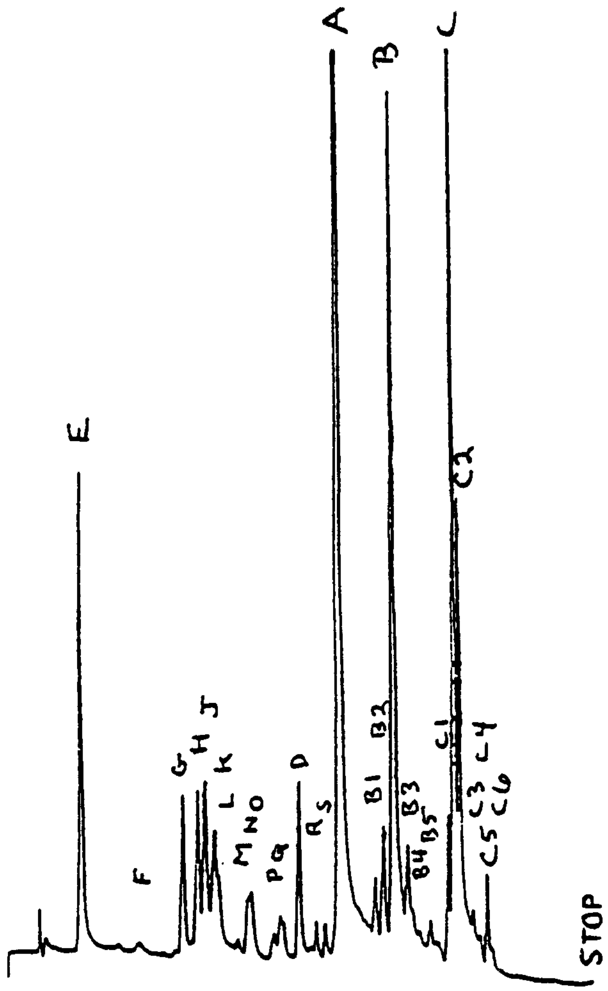


Figure